# EUROPEAN PATENT SPECIFICATION

(11) **EP 4 496 629 B1**
(45) Date of publication and mention of the grant of the patent: **10.06.2026**
(21) Application number: 23712623.0
(22) Date of filing: 20.03.2023
(51) Int. Cl.: A61P 35/00, C07D 401/14, A61K 31/53, C07C 55/10

(54) **CRYSTALLINE SALT FORM OF A SHP2 INHIBITOR**
KRISTALLINE SALZFORM EINES SHP2-INHIBITORS
FORME DE SEL CRISTALLIN D'UN INHIBITEUR DE SHP2

(30) Priority: 23.03.2022 US 202263323005 P
(43) Date of publication of application: 29.01.2025
(73) Proprietor: Array BioPharma Inc., Boulder, CO 80301 (US)
(72) Inventor: BROWN, Katie Keaton, Niwot, Colorado 80503 (US); COWDREY, Connor James, Boulder, Colorado 80301 (US); GOODWIN, Aaron Keith, Boulder, Colorado 80301 (US)
(74) Representative: Pfizer
(86) International application number: PCT/IB2023/052709
(87) International publication number: WO 2023/180898

(56) References cited:
- WO-A1-2020/201991

## Description

### BACKGROUND OF THE INVENTION

The invention relates to crystalline salts of (S)-1'-(6-((2-amino-3-chloropyridin-4-yl)thio)-1,2,4-triazin-3-yl)-1,3-dihydrospiro[indene-2,4'-piperidin]-1-amine, pharmaceutical compositions comprising the crystalline salts, and methods of using the crystalline salts in the treatment of abnormal cell growth, such as cancer, in mammals, especially humans. One crystalline salt is a crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt ("Form 1"), pharmaceutical compositions comprising succinate salt Form 1, and to methods of using succinate salt Form 1 and such compositions in the treatment of of abnormal cell growth, such as cancer, in mammals, especially humans.

The compound (S)-1'-(6-((2-amino-3-chloropyridin-4-yl)thio)-1,2,4-triazin-3-yl)-1,3-dihydrospiro[indene-2,4'-piperidin]-1-amine is a SHP2 inhibitor having the formula (I):

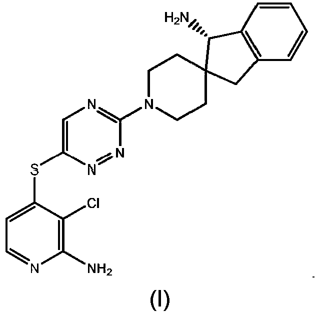

Preparation of (S)-1'-(6-((2-amino-3-chloropyridin-4-yl)thio)-1,2,4-triazin-3-yl)-1,3-dihydrospiro[indene-2,4'-piperidin]-1-amine is disclosed in International Patent Publication WO 2020/201991 (published on 8 October 2020; see also Example 1).

The present invention is defined in the appended claims.

Any references to methods of treatment in the subsequent paragraphs of this description are to be interpreted as references to the compounds, pharmaceutical compositions and medicaments of the present invention for use in method of treatment of the human (or animal) body by therapy (or diagnosis).

The preparation of a hemihydrate crystalline form of (S)-1'-(6-((2-amino-3-chloropyridin-4-yl)thio)-1,2,4-triazin-3-yl)-1,3-dihydrospiro[indene-2,4'-piperidin]-1-amine free base Form 1 is disclosed in United States Provisional Patent Application 63/169,340 (see also Example 2).

Accordingly, there remains a need for an improved form of (S)-1'-(6-((2-amino-3-chloropyridin-4-yl)thio)-1,2,4-triazin-3-yl)-1,3-dihydrospiro[indene-2,4'-piperidin]-1-amine having desirable properties, such as high crystallinity, high purity, stability, and solubility.

### Summary of the Invention

The present invention provides, in part, crystalline salt forms of (S)-1'-(6-((2-amino-3-chloropyridin-4-yl)thio)-1,2,4-triazin-3-yl)-1,3-dihydrospiro[indene-2,4'-piperidin]-1-amine. The salt forms may be useful in the treatment of abnormal cell growth. Also provided are pharmaceutical compositions, comprising the salt forms, alone or in combination with additional therapeutic agents. The present invention also provides, in part, methods for preparing such compounds, and methods of using the foregoing. This summary is provided to introduce a selection of concepts in a simplified form that are further described below in the detailed description. This summary is not intended to identify key features or essential features of the claimed subject matter, nor is it intended to be used in isolation as an aid in determining the scope of the claimed subject matter. Each of the embodiments described below can be combined with any other embodiment described herein not inconsistent with the embodiment with which it is combined.

One embodiment of the present invention provides, in part, a crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1. The succinate salt Form 1 compound may be useful in the treatment of abnormal cell growth. Also provided are pharmaceutical compositions, comprising succinate salt Form 1, alone or in combination with additional therapeutic agents. The present invention also provides, in part, methods for preparing such compounds, and methods of using the foregoing.

According to an embodiment of the invention, there is provided a crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1. The succinate salt Form 1 may be characterized by powder X-ray diffraction ("PXRD") (2θ), Raman spectrum (cm⁻¹), and/or ¹³C solid state nuclear magnetic resonance ("NMR") (ppm).

According to another embodiment of the invention, there is provided a pharmaceutical composition comprising the crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1.

According to another embodiment of the invention, there is provided the crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 for use as a medicament.

According to another embodiment of the invention, there is provided a method of treating abnormal cell growth comprising administering an effective amount of the crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1.

According to another embodiment of the invention, there is provided the crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 for use in a method of treating abnormal cell growth.

According to another embodiment of the invention, there is provided the use of the crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 for the manufacture of a medicament.

Described below are embodiments of the invention, starting with embodiment 1 (E1).

It is to be understood that both the foregoing general description and the following detailed description are exemplary and explanatory only and are not restrictive of the invention, as claimed.

### Brief Description of the Drawings

FIG. 1 shows a PXRD pattern of a crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1.
FIG. 2 shows a ¹³C ssNMR spectrum of a crystalline anhydrous form of (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1.
FIG. 3 shows a Raman spectrum of a crystalline anhydrous form of (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1.

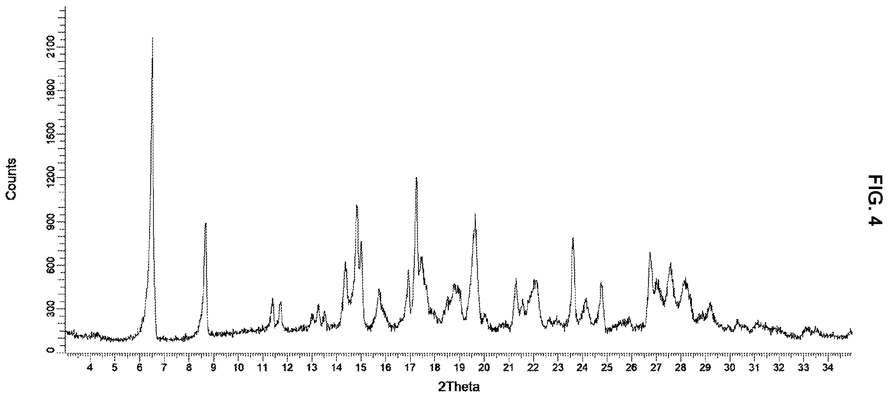

FIG. 4 shows a PXRD pattern of a crystalline form of (*S*)-1'-(6-((2-amino-3-chloropyridin-4-yl)thio)-1,2,4-triazin-3-yl)-1,3-dihydrospiro[indene-2,4'-piperidin]-1-amine phosphate salt (Pattern 1).

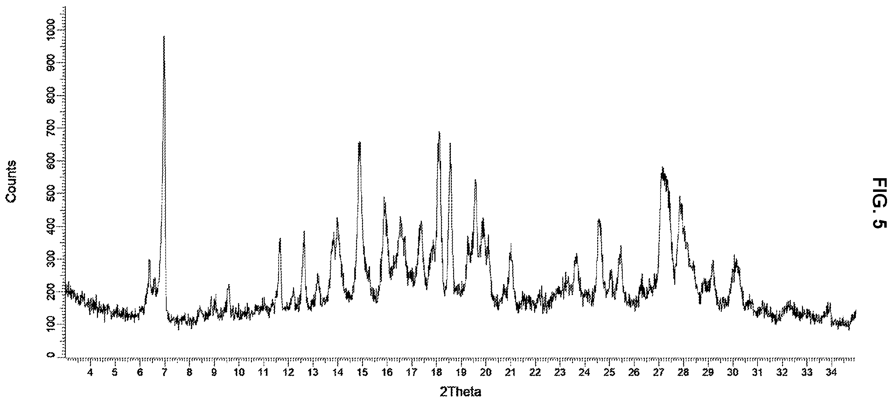

FIG. 5 shows a PXRD pattern of a crystalline form of (*S*)-1'-(6-((2-amino-3-chloropyridin-4-yl)thio)-1,2,4-triazin-3-yl)-1,3-dihydrospiro[indene-2,4'-piperidin]-1-amine phosphate salt (Pattern 2).

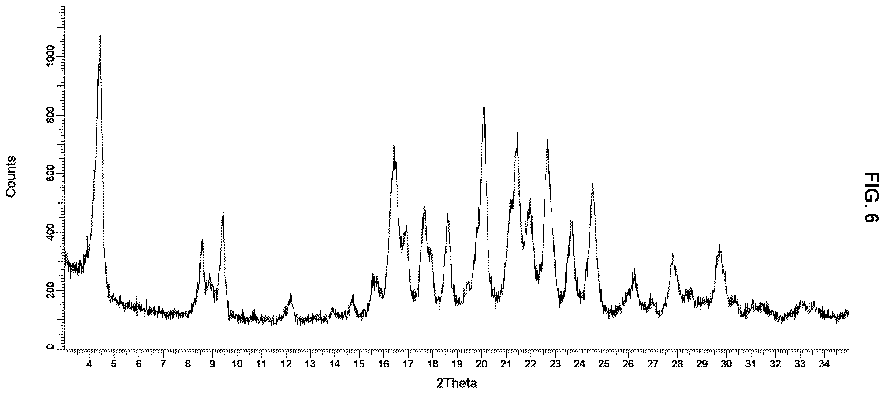

FIG. 6 shows a PXRD pattern of a crystalline form of (*S*)-1'-(6-((2-amino-3-chloropyridin-4-yl)thio)-1,2,4-triazin-3-yl)-1,3-dihydrospiro[indene-2,4'-piperidin]-1-amine fumarate salt.

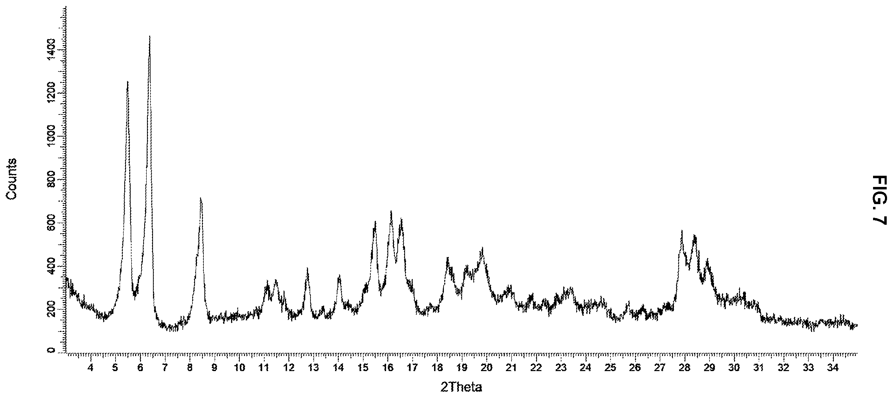

FIG. 7 shows a PXRD pattern of a crystalline form of (*S*)-1'-(6-((2-amino-3-chloropyridin-4-yl)thio)-1,2,4-triazin-3-yl)-1,3-dihydrospiro[indene-2,4'-piperidin]-1-amine tartrate salt.

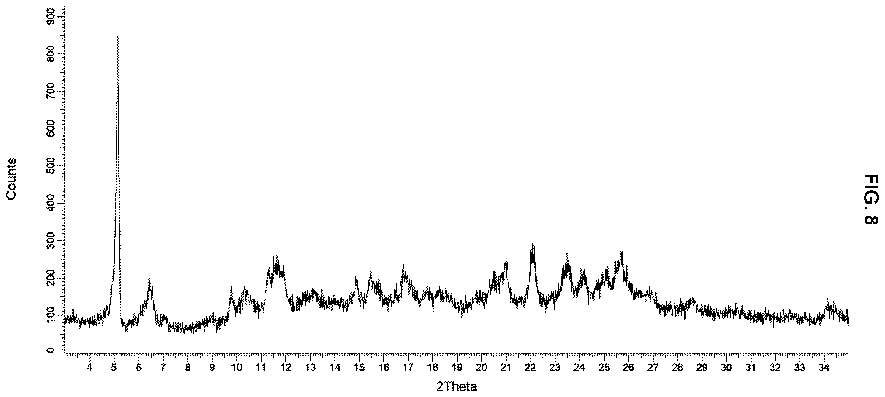

FIG. 8 shows a PXRD pattern of a crystalline form of (*S*)-1'-(6-((2-amino-3-chloropyridin-4-yl)thio)-1,2,4-triazin-3-yl)-1,3-dihydrospiro[indene-2,4'-piperidin]-1-amine hydrochloride salt.

### DETAILED DESCRIPTION OF THE INVENTION

The present invention may be understood more readily by reference to the following detailed description of the embodiments of the invention and the Examples included herein. It is to be understood that this invention is not limited to specific synthetic methods of making that may of course vary. It is to be also understood that the terminology used herein is for the purpose of describing specific embodiments only and is not intended to be limiting.

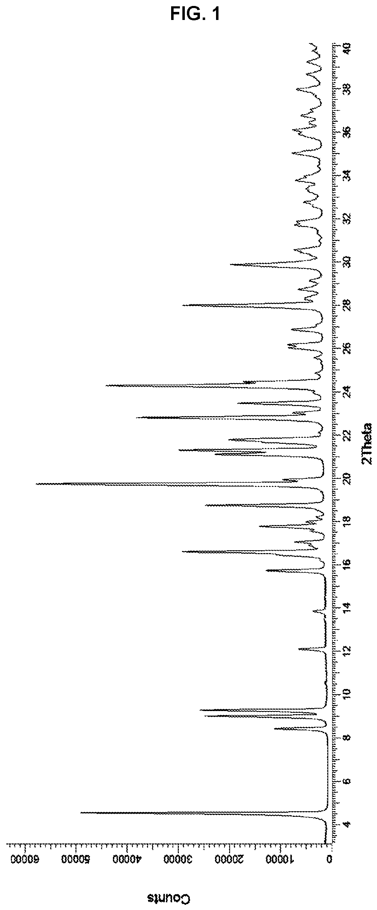

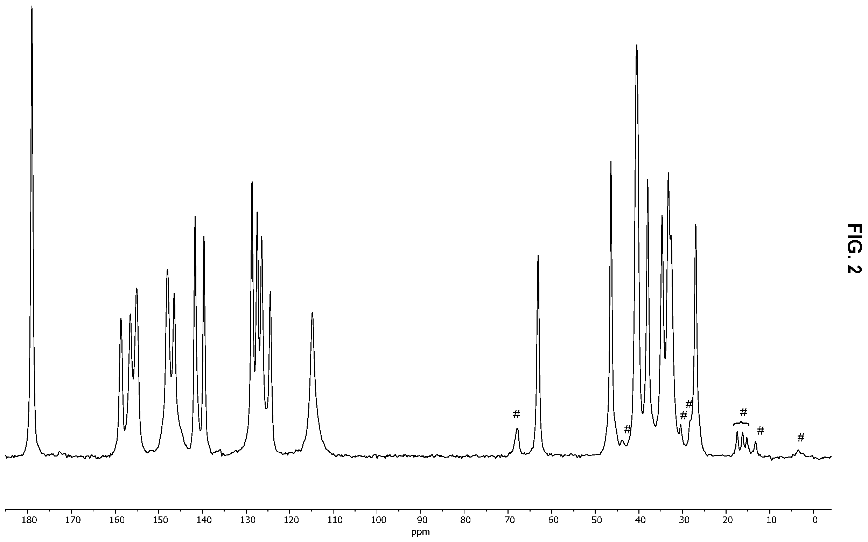

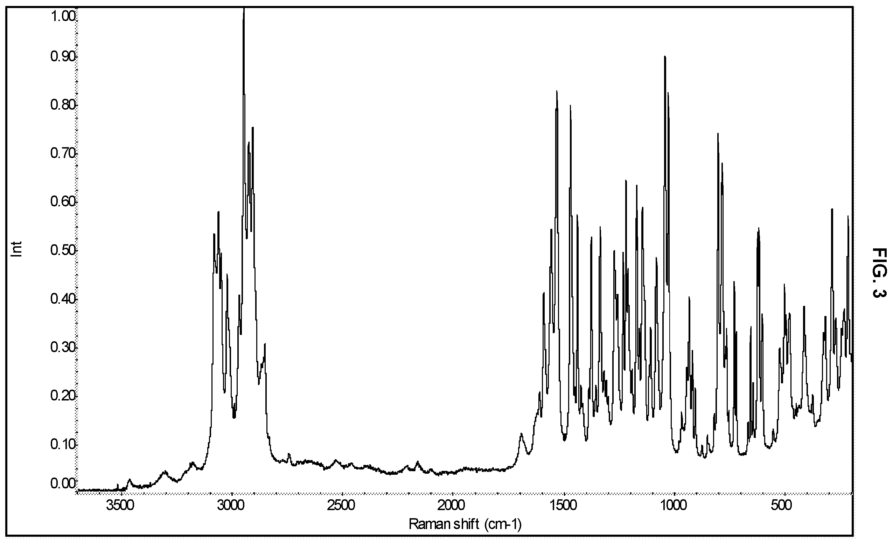

A crystalline anhydrous form of (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 has been found. Figure 1 shows a PXRD pattern of succinate salt Form 1. Figure 2 shows a ¹³C ssNMR spectrum of succinate salt Form 1. Figure 3 shows a Raman spectrum of succinate salt Form 1. As described and claimed herein, the peak positions (°2θ), wavenumber values (cm⁻¹) and resonance values (ppm), for compounds of the invention should be essentially the same. As used herein, the term "essentially the same" means that variability typical for a particular method is taken into account. For example, with reference to X-ray diffraction peak positions, the term "essentially the same" means that typical variability in peak position and intensity are taken into account. One skilled in the art will appreciate that the peak positions (2θ) will show some variability, typically as much as ± 0.2°. Further, one skilled in the art will appreciate that relative peak intensities will show inter-apparatus variability, as well as variability due to the degree of crystallinity, preferred orientation, prepared sample surface, and other factors known to those skilled in the art and should be taken as qualitative measures only. For wavenumber values, one skilled in the art will appreciate that the wavenumber values will show some variability, typically as much as ±2 cm⁻¹. For resonance values, one skilled in the art will appreciate that the resonance values will show some variability, typically as much as ±0.2 ppm.

| | |
|---|---|
| E1 | A crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt. |
| E2 | A compound of E1 characterized by: (1) a powder X-ray diffraction pattern comprising peaks at 20 values of: 19.7, 24.3, 9.3 and 16.6 ± 0.2 °2θ; (2) a Raman spectrum comprising wavenumber (cm⁻¹) values of: 1041 and 1217 cm⁻¹ ± 2 cm⁻¹; or (3) a ¹³C solid state NMR spectrum comprising a resonance (ppm) value of: 179.0 ppm ± 0.2 ppm. |
| E3 | A compound of E1 or E2 characterized by two of (1) a powder X-ray diffraction pattern comprising peaks at 2θ values of: 19.7, 24.3, 9.3 and 16.6 ± 0.2 °2θ; (2) a Raman spectrum comprising wavenumber values of: 1041 and 1217 cm⁻¹ ± 2 cm⁻¹; or (3) a ¹³C solid state NMR spectrum comprising a resonance value of: 179.0 ppm ± 0.2 ppm. |
| E4 | A compound of any one of embodiments E1 to E3 characterized by (1) a powder X-ray diffraction pattern comprising peaks at 20 values of: 19.7, 24.3, 9.3 and 16.6 ± 0.2 °2θ; (2) a Raman spectrum comprising wavenumber values of: 1041 and 1217 cm⁻¹ ±2 cm⁻¹; and (3) a ¹³C solid state NMR spectrum comprising a resonance value of: 179.0 ppm ±0.2 ppm. |
| E5 | A compound of any one of embodiments E1 to E4, having a powder X-ray diffraction pattern comprising peaks at 2θ values of: 19.7, 24.3, 9.3 and 16.6 ± 0.2 °2θ. |
| E6 | A compound of embodiment E6, having a powder X-ray diffraction pattern further comprising a peak at the 2θ value of: 9.0 ± 0.2 °2θ. |
| E7 | A compound of any one of embodiments E5 to E6, having a powder X-ray diffraction pattern further comprising a peak at the 2θ value of: 28.0 ± 0.2 °2θ. |
| E8 | A compound of any one of embodiments E5 to E7, having a powder X-ray diffraction pattern further comprising a peak at the 2θ value of: 18.8 ± 0.2 °2θ. |
| E9 | A compound of any one of embodiments E5 to E8, having a powder X-ray diffraction pattern further comprising a peak at the 2θ value of: 15.7 ± 0.2 °2θ. |
| E10 | A compound of any one of embodiments E5 to E9, having a powder X-ray diffraction pattern further comprising a peak at the 2θ value of: 12.1 ± 0.2 °2θ. |
| E11 | A compound of any one of embodiments E5 to E10, having a powder X-ray diffraction pattern further comprising peaks at 2θ values of: 4.5, 8.4, 13.9, 16.5, 17.1, 17.6, 17.8, 18.0, 19.9, 21.1, 21.3, 21.7, 21.8, 22.8, 23.0, 23.5, 24.0, 24.4, 25.6, 26.0, 26.2, 26.9, 28.3, 28.7, 29.1, 29.9, 30.5, 31.7, 31.8, 32.8, 33.4, 33.8, 35.0, 35.9, 36.1, 36.4, 36.8, 37.1, 38.0, 38.7, and 39.3 ± 0.2 °2θ. |
| E12 | A compound of any one of embodiments E5 to E11, having a PXRD essentially the same as Figure 1. |
| E13 | A compound of any one of embodiments E1 to E12, having a Raman spectrum comprising wavenumber (cm⁻¹) values of: 1041 and 1217 cm⁻¹ ± 2 cm⁻¹. |
| E14 | A compound of embodiment E13, having a Raman spectrum further comprising wavenumber (cm⁻¹) values of: 1026 and 1555 cm⁻¹ ± 2 cm⁻¹. |
| E15 | A compound of any one of embodiments E13 to E14, having a Raman spectrum essentially the same as Figure 2. |
| E16 | A compound of any one of embodiments E1 to E15, having a ¹³C solid state NMR spectrum comprising a resonance (ppm) value of: 179.0 ppm ± 0.2 ppm. |
| E17 | A compound of any one of embodiments E1 to E16, having a ¹³C solid state NMR spectrum further comprising a resonance (ppm) value of: 46.4 ppm ± 0.2 ppm. |
| E18 | A compound any one of embodiments E1 to E17, having a ¹³C solid state NMR spectrum further comprising a resonance (ppm) value of: 38.0 ppm ± 0.2 ppm. |
| E19 | A compound any one of embodiments E1 to E18, having a ¹³C solid state NMR spectrum further comprising a resonance (ppm) value of: 141.7 ppm ± 0.2 ppm. |
| E20 | A compound any one of embodiments E16 to E19, having a ¹³C solid state NMR spectrum further comprising a resonance (ppm) value of: 27.1 ppm ± 0.2 ppm. |
| E21 | A compound of any one of embodiments E16 to E20, having a ¹³C solid state NMR spectrum essentially the same as Figure 3. |
| E22 | A compound of any one of embodiments E1 to E21, which is substantially pure and free of other forms. |
| E23 | A compound of any one of embodiments E1 to E22, wherein succinate salt Form 1 is greater than 95% substantially pure. |
| E24 | A compound of any one of embodiments E1 to E23, wherein succinate salt Form 1 is greater than 97% substantially pure. |
| E25 | A compound of any one of embodiments E1 to E24, wherein succinate salt Form 1 is greater than 99% substantially pure. |
| | |

Also contemplated by the instant invention are pharmaceutical compositions comprisingsuccinate salt Form 1. A typical formulation or composition is prepared by mixing a compound described herein and a carrier or excipient. Suitable carriers and excipients are well known to those skilled in the art and are described in detail in, *e.g*., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005.

| | |
|---|---|
| E26 | A pharmaceutical composition comprising a compound of any one of embodiments E1 to E25 and at least one pharmaceutically acceptable excipient. |

The compositions of this invention may be in a variety of forms. These include, for example, liquid, semi-solid and solid dosage forms, such as liquid solutions (e.g., injectable and infusible solutions), dispersions or suspensions, tablets, capsules, pills, powders, liposomes and suppositories. The form depends on the intended mode of administration and therapeutic application.

Typical compositions are in the form of injectable or infusible solutions, such as compositions similar to those used for passive immunization of humans with antibodies in general. One mode of administration is parenteral (e.g., intravenous, subcutaneous, intraperitoneal, intramuscular). In another embodiment, the compound is administered by intravenous infusion or injection. In yet another embodiment, the compound is administered by intramuscular or subcutaneous injection.

Oral administration of a solid dosage form may be, for example, presented in discrete units, such as hard or soft capsules, pills, cachets, lozenges, or tablets, each containing a predetermined amount of at least one compound of the invention. In another embodiment, the oral administration may be in a powder or granule form. In another embodiment, the oral dosage form is sub-lingual, such as, for example, a lozenge. In such solid dosage forms, the compounds of the invention are ordinarily combined with one or more adjuvants. Such capsules or tablets may comprise a controlled release formulation. In the case of capsules, tablets, and pills, the dosage forms also may comprise buffering agents or may be prepared with enteric coatings.

Other excipients and modes of administration known in the pharmaceutical art may also be used. Pharmaceutical compositions of the invention may be prepared by any of the well-known techniques of pharmacy, such as effective formulation and administration procedures. The above considerations in regard to effective formulations and administration procedures are well known in the art and are described in standard textbooks. Formulation of drugs is discussed in, for example, Ansel (supra) and Gennaro (supra).

The present invention also contemplates the crystalline anhydrous form of (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 for use as a medicament.

| | |
|---|---|
| E27 | The compound of any one of embodiments E1 to E26 for use as a medicament. |

The present invention also contemplates a method of treatment comprising administering the crystalline anhydrous form of (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 according to any of the embodiments described herein. In particular, the method of treatment is to a subject in need thereof. In most embodiments, the subject is a mammal. In some embodiments, the subject is a human.

| | |
|---|---|
| E28 | A method for treating abnormal cell growth, comprising administering an effective amount to a subject in need thereof a therapeutically effective amount of the compound of any one of embodiments E1 to E26. |
| E29 | A method for treating abnormal cell growth in a mammal comprising administering an effective amount to the mammal a therapeutically effective amount of the compound of any one of embodiments E1 to E26. |
| E30 | The method of embodiment E29, wherein the mammal is human. |
| E31 | The method of any one of embodiments E28 to E30, wherein the abnormal cell growth is cancer. |

The present invention also contemplates the crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 for use in the treatment of abnormal cell growth.

| | |
|---|---|
| E32 | The compound of any one of embodiments E1 to E26 for use in the treatment of abnormal cell growth. |
| E33 | The compound of any one of embodiments E1 to E26 for use in the treatment of abnormal cell growth in a mammal. |
| E34 | The compound of any one of embodiments E1 to E26 for use in the treatment of abnormal cell growth in a human. |
| E35 | The compound of any one of embodiments E32 to E34, wherein the abnormal cell growth is cancer. |

The present invention also contemplates the use of crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 for the manufacture of a medicament.

| | |
|---|---|
| E36 | Use of the compound of any one of embodiments E1 to E26 for the manufacture of a medicament. |
| E37 | Use of the compound of any one of embodiments E1 to E26 for the manufacture of a medicament for the treatment of abnormal cell growth. |
| E38 | Use of the compound of any one of embodiments E1 to E26 for the manufacture of a medicament for the treatment of abnormal cell growth in a mammal. |
| E39 | Use of the compound of any one of embodiments E1 to E26 for the manufacture of a medicament for the treatment of abnormal cell growth in a human. |
| E40 | The use of any one of embodiments E36 to E39, wherein the abnormal cell growth is cancer. |

In frequent embodiments of the methods provided herein, the abnormal cell growth is cancer. "Cancer", as used herein, means the physiological condition in mammals that is typically characterized by abnormal or unregulated cell growth. Cancer includes solid tumors named for the type of cells that form them, cancer of blood, bone marrow, or the lymphatic system. Examples of solid tumors include sarcomas and carcinomas. Cancers of the blood include, but are not limited to, leukemia, lymphoma and myeloma. Cancer also includes primary cancer that originates at a specific site in the body, a metastatic cancer that has spread from the place in which it started to other parts of the body, a recurrence from the original primary cancer after remission, and a second primary cancer that is a new primary cancer in a person with a history of previous cancer of a different type from the latter one.

The methods provided result in one or more of the following effects: (1) inhibiting cancer cell proliferation; (2) inhibiting cancer cell invasiveness; (3) inducing apoptosis of cancer cells; (4) inhibiting cancer cell metastasis; or (5) inhibiting angiogenesis.

The treatment achieved by crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 is defined by reference to any of the following: partial response (PR), complete response (CR), overall response (OR), progression free survival (PFS), disease free survival (DFS) and overall survival (OS). PFS, also referred to as "Time to Tumor Progression" indicates the length of time during and after treatment that the cancer does not grow and includes the amount of time patients have experienced a CR or PR, as well as the amount of time patients have experienced stable disease (SD). DFS refers to the length of time during and after treatment that the patient remains free of disease. OS refers to a prolongation in life expectancy as compared to naïve or untreated subjects or patients. In some embodiments, response to a combination of the invention is any of PR, CR, PFS, DFS, OR or OS that is assessed using Response Evaluation Criteria in Solid Tumors (RECIST) 1.1 response criteria.

| | |
|---|---|
| E41 | The method, compound or use of any one of embodiments E31, E35 or E40, wherein the cancer is selected from the group consisting of melanoma, juvenile myelomoncytic leukemias, neuroblastoma, Philadelphia chromosome positive chronic myeloid leukemia, Philadelphia chromosome positive acute lymphoblastic leukemias, acute myeloid leukemias, myeloproliferative neoplasms (such as Polycythemia Vera, Essential Thrombocythemia and Primary Myelofibrosis), breast cancer, lung cancer, liver cancer, colorectal cancer, esophageal cancer, gastric cancer, squamous-cell carcinoma of the head and neck, glioblastoma, anaplastic large-cell lymphoma, thyroid carcinoma, and spitzoid neoplasms. |
| E42 | The method, compound or use of embodiment E41, wherein the cancer is melanoma. |
| E43 | The method, compound or use of embodiment E41, wherein the cancer is juvenile myelomoncytic leukemias. |
| E44 | The method, compound or use of embodiment E41, wherein the cancer is neuroblastoma. |
| E45 | The method, compound or use of embodiment E41, wherein the cancer is Philadelphia chromosome positive chronic myeloid leukemia. |
| E46 | The method, compound or use of embodiment E41, wherein the cancer is Philadelphia chromosome positive acute lymphoblastic leukemia. |
| E47 | The method, compound or use of embodiment E41, wherein the cancer is acute myeloid leukemia. |
| E48 | The method, compound or use of embodiment E41, wherein the cancer is myeloproliferative neoplasms (such as Polycythemia Vera, Essential Thrombocythemia and Primary Myelofibrosis). |
| E49 | The method, compound or use of embodiment E48, wherein the cancer is Polycythemia Vera. |
| E50 | The method, compound or use of embodiment E48, wherein the cancer is Essential Thrombocythemia. |
| E51 | The method, compound or use of embodiment E48, wherein the cancer is Primary Myelofibrosis. |
| E52 | The method, compound or use of embodiment E41, wherein the cancer is breast cancer. |
| E53 | The method, compound or use of embodiment E41, wherein the cancer is lung cancer. |
| E54 | The method, compound or use of embodiment E41, wherein the cancer is liver cancer. |
| E55 | The method, compound or use of embodiment E41, wherein the cancer is colorectal cancer. |
| E56 | The method, compound or use of embodiment E41, wherein the cancer is esophageal cancer. |
| E57 | The method, compound or use of embodiment E41, wherein the cancer is gastric cancer. |
| E58 | The method, compound or use of embodiment E41, wherein the cancer is squamous-cell carcinoma of the head and neck. |
| E59 | The method, compound or use of embodiment E41, wherein the cancer is glioblastoma. |
| E60 | The method, compound or use of embodiment E41, wherein the cancer is anaplastic large-cell lymphoma. |
| E61 | The method, compound or use of embodiment E41, wherein the cancer is thyroid carcinoma. |
| E62 | The method, compound or use of embodiment E41, wherein the cancer is spitzoid neoplasms. |
| E63 | The method, compound or use of any one of embodiments E31, E35 or E40, wherein the cancer is selected from the group consisting of non-small cell lung cancer ("NSCLC"), colon cancer, esophageal cancer, rectal cancer, juvenile myelomonocytic leukemia ("JMML"), breast cancer, melanoma, and pancreatic cancer. |
| E64 | The method, compound or use of embodiment E63, wherein the cancer is NSCLC. |
| E65 | The method, compound or use of embodiment E63, wherein the cancer is colon cancer. |
| E66 | The method, compound or use of embodiment E63, wherein the cancer is esophageal cancer. |
| E67 | The method, compound or use of embodiment E63, wherein the cancer is rectal cancer. |
| E68 | The method, compound or use of embodiment E63, wherein the cancer is JMML. |
| E69 | The method, compound or use of embodiment E63, wherein the cancer is breast cancer. |
| E70 | The method, compound or use of embodiment E63, wherein the cancer is melanoma. |
| E71 | The method, compound or use of embodiment E63, wherein the cancer is pancreatic cancer. |
| E72 | The method, compound or use of any one of embodiments E31, E35 or E40, wherein the cancer is selected from the group consisting of ALK-positive NSCLC, ROS1-positive NSCLC, BRAF V600E mutation colorectal cancer, RAS-mutant solid tumors, NF1-mutant solid tumors, and BRAF class 3-mutant solid tumors. |
| E73 | The method, compound or use of embodiment E72, wherein the cancer is ALK-positive NSCLC. |
| E74 | The method, compound or use of embodiment E73, wherein the cancer being treated is with prior lorlatinib treatment and with prior platinum-based chemotherapy treatment. |
| E75 | The method, compound or use of embodiment E73, wherein the cancer being treated is with prior lorlatinib treatment and without prior platinum-based chemotherapy treatment. |
| E76 | The method, compound or use of embodiment E73, wherein the cancer being treated is without prior lorlatinib treatment. |
| E77 | The method, compound or use of embodiment E72, wherein the cancer is ROS1-positive NSCLC. |
| E78 | The method, compound or use of embodiment E72, wherein the cancer is BRAF V600E mutation colorectal cancer. |
| E79 | The method, compound or use of embodiment E78, wherein the cancer being treated is resistant to BRAF inhibitor ("BRAFi") plus epidermal growth factor receptor inhibitor ("EGFRi") treatment. |
| E80 | The method, compound or use of embodiment E78, wherein the cancer being treated is refractory BRAFi plus EGFRi treatment. |
| E81 | The method, compound or use of embodiment E78, wherein the cancer being treated is with no prior BRAFi plus EGFRi treatment. |
| E82 | The method, compound or use of embodiment E72, wherein the cancer is RAS-mutant solid tumors. |
| E83 | The method, compound or use of embodiment E81, wherein the cancer being treated has received prior standard of care. |
| E84 | The method, compound or use of embodiment E72, wherein the cancer is NF1-mutant solid tumors. |
| E85 | The method, compound or use of embodiment E84, wherein the cancer being treated has received prior standard of care. |
| E86 | The method, compound or use of embodiment E72, wherein the cancer is BRAF class 3-mutant solid tumors. |
| E87 | The method, compound or use of embodiment E86, wherein the BRAF class 3-mutation is selected from one or more of the following amino acid substitutions in human BRAF: |
| | D287H; P367R; V459L; G466V; G466E; G466A; S467L; G469E; N581S; N581I; D594N; D594G; D594A; D594H; F595L; G596D; G596R and A762E. |
| E88 | The method, compound or use of embodiments E86 or E87, wherein the cancer being treated has received prior standard of care. |
| E89 | The method, compound or use of any one of embodiments E31, E35 or E40, wherein the cancer is a KRAS mutation cancer. |
| E90 | The method, compound or use of embodiment E89, wherein the cancer is selected from a KRAS^{G12A} mutation, a KRAS^{G12C} mutation, a KRAS^{G12D} mutation, a KRAS^{G12F} mutation, a KRAS^{G12I} mutation, a KRAS^{G12L} mutation, a KRAS^{G12R} mutation, a KRAS^{G12S} mutation, a KRAS^{G12V} mutation, and a KRAS^{G12Y} mutation. |

The present invention also contemplates the crystalline anhydrous form of (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 is used in combination with an additional therapeutic compound.

The administration of two or more compounds "in combination" means that all of the compounds are administered closely enough in time to affect treatment of the subject. The two or more compounds may be administered simultaneously or sequentially, via the same or different routes of administration, on same or different administration schedules and with or without specific time limits depending on the treatment regimen. Additionally, simultaneous administration may be carried out by mixing the compounds prior to administration or by administering the compounds at the same point in time but as separate dosage forms at the same or different site of administration. Examples of "in combination" include, but are not limited to, "concurrent administration," "co-administration," "simultaneous administration," "sequential administration" and "administered simultaneously".

The crystalline anhydrous form of (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 and the one or more other therapeutic agents may be administered as a fixed or non-fixed combination of the active ingredients. The term "fixed combination" means the crystalline anhydrous form of (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 and the one or more therapeutic agents, are both administered to a subject simultaneously in a single composition or dosage. The term "non-fixed combination" means that the crystalline anhydrous form of (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 and the one or more therapeutic agents are formulated as separate compositions or dosages such that they may be administered to a subject in need thereof simultaneously or at different times with variable intervening time limits, wherein such administration provides effective levels of the two or more compounds in the body of the subject.

| | |
|---|---|
| E91 | The method, compound or use of any one of embodiments E27 to E90, wherein the compound of any one of embodiments E1 to E26 is used in combination with an additional therapeutic compound. |
| E92 | The method, compound or use of embodiment E91, wherein the additional therapeutic compound is selected from the group consisting of lorlatinib, binimietinib, cetuximab, and encorafenib. |
| E93 | The method, compound or use of embodiment E91 or E92, wherein the additional therapeutic compound is lorlatinib. |
| E94 | The method, compound or use of embodiment E91 or E92, wherein the additional therapeutic compound is binimetinib. |
| E95 | The method, compound or use of embodiment E91 or E92, wherein the additional therapeutic compound is cetuximab and encorafenib. |
| E96 | The method, compound or use of embodiment E91 or E92, wherein the additional therapeutic compound is cetuximab. |
| E97 | The method, compound or use of embodiment E91 or E92, wherein the additional therapeutic compound is encorafenib. |

### Definitions

As used herein, the singular form "a", "an", and "the" include plural references unless indicated otherwise. For example, "a" substituent includes one or more substituents.

The term "about" means having a value falling within an accepted standard of error of the mean, when considered by one of ordinary skill in the art.

The term "crystalline" as used herein, means having a regularly repeating arrangement of molecules or external face planes. Crystalline forms may differ with respect to thermodynamic stability, physical parameters, x-ray structure and preparation processes.

The invention described herein may be suitably practiced in the absence of any element(s) not specifically disclosed herein. Thus, for example, in each instance herein any of the terms "comprising", "consisting essentially of", and "consisting of" may be replaced with either of the other two terms.

The term "substantially pure" means a particular crystalline form includes less than 10%, preferably less than 5%, preferably less than 3%, preferably less than 1% by weight of any other physical forms of the compound.

"Pharmaceutical composition", as used herein, means the crystalline anhydrous form of (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 according to any of the embodiments described herein as an active ingredient, and at least one pharmaceutically acceptable excipient.

"Pharmaceutically acceptable carrier", as used herein, means a carrier or diluent that does not cause significant irritation to an organism and does not abrogate the biological activity and properties of the administered compound.

The term "excipient" is used herein to describe any ingredient other than the crystalline anhydrous form of (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1. The choice of excipient will to a large extent depend on factors such as the mode of administration, the effect of the excipient on solubility and stability, and the nature of the dosage form. Excipient includes any and all solvents, dispersion media, coatings, antibacterial and antifungal agents, isotonic and absorption delaying agents, carriers, diluents and the like that are physiologically compatible. Examples of excipients include one or more of water, saline, phosphate buffered saline, dextrose, glycerol, ethanol and the like, as well as combinations thereof, and may include isotonic agents, for example, sugars, sodium chloride, or polyalcohols such as mannitol, or sorbitol in the composition. Examples of excipients also include various organic solvents (such as hydrates and solvates). The pharmaceutical compositions may, if desired, contain additional excipients such as flavorings, binders/binding agents, lubricating agents, disintegrants, sweetening or flavoring agents, coloring matters or dyes, and the like. For example, for oral administration, tablets containing various excipients, such as citric acid may be employed together with various disintegrants such as starch, alginic acid and certain complex silicates and with binding agents such as sucrose, gelatin and acacia. Examples, without limitation, of excipients include calcium carbonate, calcium phosphate, various sugars and types of starch, cellulose derivatives, gelatin, vegetable oils and polyethylene glycols. Additionally, lubricating agents such as magnesium stearate, sodium lauryl sulfate and talc are often useful for tableting purposes. Solid compositions of a similar type may also be employed in soft and hard filled gelatin capsules. Non-limiting examples of excipients, therefore, also include lactose or milk sugar and high molecular weight polyethylene glycols. When aqueous suspensions or elixirs are desired for oral administration the active compound therein may be combined with various sweetening or flavoring agents, coloring matters or dyes and, if desired, emulsifying agents or suspending agents, together with additional excipients such as water, ethanol, propylene glycol, glycerin, or combinations thereof.

Examples of excipients also include pharmaceutically acceptable substances such as wetting agents or minor amounts of auxiliary substances such as wetting or emulsifying agents, preservatives, or buffers, which enhance the shelf life or effectiveness of the compound.

As used herein, an "effective dosage" or "effective amount" of drug, compound or pharmaceutical composition is an amount sufficient to affect any one or more beneficial or desired, including biochemical, histological and/or behavioral symptoms, of the disease, its complications and intermediate pathological phenotypes presenting during development of the disease. For therapeutic use, a "therapeutically effective amount" refers to that amount of a compound being administered that will relieve to some extent one or more of the symptoms of the disorder being treated. In reference to the treatment of cancer, a therapeutically effective amount refers to that amount which has the effect of (1) reducing the size of the tumor, (2) inhibiting (that is, slowing to some extent, preferably stopping) tumor metastasis, (3) inhibiting to some extent (that is, slowing to some extent, preferably stopping) tumor growth or tumor invasiveness, (4) relieving to some extent (or, preferably, eliminating) one or more signs or symptoms associated with the cancer, (5) decreasing the dose of other medications required to treat the disease, and/or (6) enhancing the effect of another medication, and/or (7) delaying the progression of the disease in a patient.

"Mammal", as used herein, means a warm-blooded animal that has or is at risk of developing a disease described herein and includes, but is not limited to, guinea pigs, dogs, cats, rats, mice, hamsters, and primates, including humans.

"Subject", as used herein, means a human or animal subject. In certain embodiments, the subject is a mammal. In certain preferred embodiments, the subject is a human.

"Treat" or "treating", as used herein, means to administer crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 to a subject having the condition to be treated to achieve at least one positive therapeutic effect. For example, treating cancer means to administer crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 to a subject having cancer, or diagnosed with cancer, to achieve at least one positive therapeutic effect, such as, for example, reduced number of cancer cells, reduced tumor size, reduced rate of cancer cell infiltration into peripheral organs, or reduced rate of tumor metastases or tumor growth, reversing, alleviating, inhibiting the progress of, or preventing the disorder or condition to which such term applies, or one or more symptoms of such disorder or condition. The term "treatment", as used herein, unless otherwise indicated, means the act of treating as "treating" is defined immediately above. The term "treating" also includes adjuvant and neo-adjuvant treatment of a subject.

### EXAMPLES

The examples and preparations provided below further illustrate and exemplify particular aspects and embodiments of the invention. It is to be understood that the scope of the present invention is not limited by the scope of the following examples. The compounds and intermediates described herein were named using the naming convention provided with ChemDraw Professional, Version 19.0.0.22 (Perkin Elmer Informatics, Inc., Waltham, Mass.).

### Example 1

### Amorphous (S)-1'-(6-((2-amino-3-chloropyridin-4-yl)thio)-1,2,4-triazin-3-yl)-1,3-dihydrospiro[indene-2,4'-piperidin]-1-amine free base

(*R*)-*N*-((*S*)-1'-(6-((2-Amino-3-chloropyridin-4-yl)thio)-1,2,4-triazin-3-yl)-1,3-dihydrospiro[indene-2,4'-piperidin]-1-yl)-2-methylpropane-2-sulfinamide (20 mg, 0.037 mmol) was diluted with dioxane (1 mL), followed by the addition of HCl (92 µL, 0.37 mmol). After stirring for 30 minutes, the reaction was diluted with dichloromethane ("DCM") and saturated aqueous sodium bicarbonate. After stirring the mixture for 10 minutes, the layers were separated, and the DCM was dried over MgSO₄, filtered and concentrated. The material was purified on silica gel eluting with 20% methanol/ethyl acetate to afford amorphous (*S*)-1'-(6-((2-amino-3-chloropyridin-4-yl)thio)-1,2,4-triazin-3-yl)-1,3-dihydrospiro[indene-2,4'-piperidin]-1-amine free base (15 mg, 0.034 mmol, 93% yield). ¹H NMR (400 MHz, CDCl₃) δ 8.2 (s, 1H), 7.76 (d, 1H, J = 5.2 Hz), 7.2-7.35 (m, 4H), 6.15 (d, 1H, J = 5.2 Hz), 4.92 (br, 2H), 4.78 (br, 1H), 4.01 (s, 1H), 3.37 (m, 2H), 3.14 (d, 1H, J = 15.6 Hz), 2.78 (d, 1H, J = 15.6), 1.3-1.91 (m, 7H); *m*/*z* (esi/APCI) M⁺1 = 440.1.

### Example 2

### Crystalline (S)-1'-(6-((2-amino-3-chloropyridin-4-yl)thio)-1,2,4-triazin-3-yl)-1,3-dihydrospiro[indene-2,4'-piperidin]-1-amine free base hemihydrate (Form 1)

(*S*)-1,3-Dihydrospiro[indene-2,4'-piperidin]-1-amine dihydrochloride (3.89 Kg) and 3,6-dibromo-1,2,4-triazine (3.71 Kg) were reacted with triethylamine (6.45 Kg) in 1,4-dioxane (34.3 Kg) at 20-30 °C to provide (*S*)-1'-(6-bromo-1,2,4-triazin-3-yl)-1,3-dihydrospiro[indene-2,4'-piperidin]-1-amine in situ. (*S*)-1'-(6-Bromo-1,2,4-triazin-3-yl)-1,3-dihydrospiro[indene-2,4'-piperidin]-1-amine was reacted with sodium 2-amino-3-chloropyridine-4-thiolate (3.08 Kg) and triethylamine (6.45 Kg) in 1,4-dioxane (2.05 Kg) at 20-30 °C, and the mixture was heated to 70-75 °C for about 12.5 hours to provide (*S*)-1'-(6-((2-amino-3-chloropyridin-4-yl)thio)-1,2,4-triazin-3-yl)-1,3-dihydrospiro[indene-2,4'-piperidin]-1-amine. The mixture was cooled to about 15-30 °C. Celite^{®} (1.95 Kg) was added to the reaction at 15-30 °C and stirred for about 1 hour, and the slurry was filtered and rinsed with 1,4-dioxane. A solvent swap was performed to replace 1,4-dioxane with tetrahydrofuran. Crude (*S*)-1'-(6-((2-amino-3-chloropyridin-4-yl)thio)-1,2,4-triazin-3-yl)-1,3-dihydrospiro[indene-2,4'-piperidin]-1-amine was purified by chromatography on silica gel (pre-treated with isopropyl alcohol) using mixtures of dichloromethane, methanol, ethanol, *n-*hexane and triethylamine as the eluent. After chromatography, the product (1.57 Kg) was combined with methanol (24.0 Kg) and water (1.6 Kg) at 15-30 °C. The mixture was heated to 40 °C and reduced pressure was applied until about 22-25 L of mixture remained. Additional methanol (24.0 Kg) and water (1.6 Kg) were added at 15-30 °C. The mixture was heated to 40 °C and reduced pressure was applied until about 22-25 L of mixture remained. Additional water (15.70 Kg) was added at 15-30 °C. The mixture was stirred at 15-25 °C for 12 hours to induce crystallization. Product is collected via filtration, and the filter cake was rinsed with a methanol (3.7 Kg) and water (1.6 Kg) mixture to afford purified crystalline (S)-1'-(6-((2-amino-3-chloropyridin-4-yl)thio)-1,2,4-triazin-3-yl)-1,3-dihydrospiro[indene-2,4'-piperidin]-1-amine free base hemihydrate (Form 1) (1.39 Kg).

Powder X-ray diffraction analysis was conducted using a Bruker AXS D8 Endeavor diffractometer equipped with a Copper (Cu) radiation source. The divergence slit was set at 15 mm continuous illumination. Diffracted radiation was detected by a PSD-Lynx Eye detector, with the detector PSD opening set at 2.99 degrees. The X-ray tube voltage and amperage were set to 40 kV and 40 mA respectively. Data was collected in the Theta-Theta goniometer at the Cu wavelength (CuK_{α̅} = 1.5418 A) from 3.0 to 40.0 degrees 2-Theta using a step size of 0.00998 degrees and a step time of 1.0 second. The anti-scatter screen was set to a fixed distance of 3.0 mm. Samples were rotated at 15/min during collection. Samples were prepared by placing them in a silicon low background sample holder and rotated during collection. Data were collected using Bruker DIFFRAC Plus software and analysis was performed by EVA diffract plus software. The PXRD data file was not processed prior to peak searching. Using the peak search algorithm in the EVA software, peaks selected with a threshold value of 1 were used to make preliminary peak assignments. To ensure validity, adjustments were manually made; the output of automated assignments was visually checked, and peak positions were adjusted to the peak maximum. Peaks with relative intensity of ≥ 3% were generally chosen. Typically, the peaks which were not resolved or were consistent with noise were not selected. A typical error associated with the peak position from PXRD stated in USP up to +/- 0.2° 2-Theta (USP-941).

A PXRD peak list and relative intensity data for the compound of Example 2, free base hemihydrate Form 1 (2-Theta °) is provided in Table 1 below:

**Table 1**

| **Angle (2 theta** °) ± **0.2 °2**θ | **Relative Intensity** (%) | **Angle (2 theta** °) ± **0.2 °2**θ | **Relative Intensity** (%) |
|---|---|---|---|
| 5.4 | 6.8 | 26.8 | 13.9 |
| 10.9 | 60.1 | 27.1 | 51.2 |
| 12.1 | 5.2 | 27.3 | 50.9 |
| 13.5 | 3.4 | 27.8 | 11.6 |
| 14.0 | 40.2 | 28.2 | 71.3 |
| 15.4 | 54.9 | 29.3 | 16.6 |
| 15.5 | 44.5 | 29.5 | 41.5 |
| 16.0 | 61.8 | 30.0 | 5.6 |
| 16.4 | 23.1 | 31.5 | 12.6 |
| 17.2 | 49.8 | 31.9 | 3.9 |
| 17.7 | 16.5 | 32.2 | 9.9 |
| 19.0 | 19.1 | 33.2 | 12.1 |
| 19.3 | 45.5 | 33.3 | 10.0 |
| 19.6 | 41.2 | 33.9 | 4.5 |
| 19.7 | 41.5 | 34.1 | 5.7 |
| 20.5 | 39.6 | 34.7 | 7.4 |
| 21.8 | 33.4 | 35.2 | 11.7 |
| 22.2 | 15.0 | 35.4 | 12.6 |
| 22.5 | 100.0 | 36.1 | 5.9 |
| 23.5 | 5.5 | 36.9 | 3.3 |
| 24.3 | 18.4 | 37.2 | 5.0 |
| 25.1 | 68.5 | 37.5 | 3.2 |
| 25.4 | 5.3 | 38.0 | 5.6 |
| 25.7 | 12.5 | 38.6 | 3.5 |
| 26.0 | 7.2 | 39.0 | 4.5 |

The characteristic PXRD peak list and relative intensity data for the compound of Example 2, free base hemihydrate Form 1 (2-Theta °) is provided in Table 2 below:

**Table 2**

| **Angle (° 2-Theta)** ± **0.2 °2**θ | **Relative Intensity** (%) |
|---|---|
| 10.9 | 60.1 |
| 16.0 | 61.8 |
| 14.0 | 40.2 |
| 15.4 | 54.9 |
| 15.5 | 44.5 |
| 25.1 | 68.5 |
| 20.5 | 39.6 |
| 29.5 | 41.5 |

Solid-state NMR ("ssNMR") analysis was conducted on a cross-polarization magic angle spinning ("CPMAS") probe positioned into a Bruker-BioSpin Avance III 500 MHz (¹H frequency) NMR spectrometer. Material was packed into a 4 mm ZrO₂ rotor. A magic angle spinning rate of 14.0 kHz was used. Spectra were collected at ambient temperature (temperature uncontrolled).

¹³C ssNMR spectrum were collected using a proton decoupled CPMAS experiment. A phase modulated proton decoupling field of 80-100 kHz was applied during spectral acquisition. The cross-polarization contact time was set to 2 ms and the recycle delay to 11.4 seconds. The number of scans was adjusted to obtain an adequate signal to noise ratio. The ¹³C chemical shift scale was referenced using a ¹³C CPMAS experiment on an external standard of crystalline adamantane, setting its up-field resonance to 29.5 ppm.

Automatic peak picking was performed using Bruker-BioSpin TopSpin version 3.6 software. Generally, a threshold value of 5% relative intensity was used for preliminary peak selection. The output of the automated peak picking was visually checked to ensure validity and adjustments were manually made if necessary. Although specific solid-state NMR peak values are reported herein there does exist a range for these peak values due to differences in instruments, samples, and sample preparation. This is common practice in the art of solid-state NMR because of the variation inherent in peak positions. A typical variability for a ¹³C chemical shift x-axis value is on the order of plus or minus 0.2 ppm for a crystalline solid. The solid-state NMR peak heights reported herein are relative intensities. Solid-state NMR intensities can vary depending on the actual setup of the CPMAS experimental parameters and the thermal history of the sample.

For crystalline (S)-1'-(6-((2-amino-3-chloropyridin-4-yl)thio)-1,2,4-triazin-3-yl)-1,3-dihydrospiro[indene-2,4'-piperidin]-1-amine free base hemihydrate (Form 1), five characteristic peaks were identified: ¹³C chemical shifts at 44.4, 47.7, 149.2, 36.6, and 26.9 ± 0.2 ppm. The characteristic peaks were chosen because they have high intensity, a narrow peak shape, are unique to the solid form, and are likely to not be obscured in drug product spectra.

The ¹³C ssNMR spectrum of Example 2, free base hemihydrate Form 1 (ppm) is provided in Table 3 below:

**Table 3**

| **¹³C Chemical Shift (ppm) ± 0.2 ppm** | **Relative Intensity** (%) |
|---|---|
| 26.9 | 51 |
| 33.8 | 39 |
| 35.0 | 59 |
| 36.6 | 37 |
| 39.0 | 82 |
| 39.4 | 67 |
| 40.2 | 67 |
| 41.3 | 38 |
| 43.4 | 81 |
| 44.4 | 74 |
| 47.7 | 63 |
| 66.0 | 49 |
| 68.9 | 38 |
| 109.0 | 93 |
| 124.5 | 100 |
| 127.0 | 41 |
| 127.9 | 70 |
| 128.9 | 51 |
| 129.8 | 43 |
| 139.7 | 88 |
| 140.9 | 48 |
| 145.1 | 57 |
| 145.4 | 79 |
| 146.5 | 39 |
| 149.2 | 49 |
| 154.5 | 48 |
| 155.9 | 64 |
| 158.9 | 38 |

Raman spectra were collected using a Thermo Scientific iS50 FT-Raman accessory attached to the FT-IR bench. A CaF2 beam splitter is utilized in the FT-Raman configuration. The spectrometer is equipped with a 1064 nm diode laser and a room temperature InGaAs detector. Prior to data acquisition, instrument performance and calibration verifications were conducted using polystyrene. Samples were analyzed in glass NMR tubes, as tablets or in a suitable sample holder held static during data collection. The spectra were collected using between 0.1 and 0.5 W of laser power and 512 co-added scans. The collection range was 3700-100 cm⁻¹. The API spectra were recorded using 2 cm⁻¹ resolution, and Happ-Genzel apodization was utilized for all of the spectra. Multiple spectra were recorded, and the reported spectrum is representative of two spots.

The intensity scale was normalized to 1 prior to peak picking. Peaks were manually identified using the Thermo Nicolet Omnic 9.7.46 software. Peak position was picked at the peak maximum, and peaks were only identified as such, if there was a slope on each side; shoulders on peaks were not included. For neat free base hemihydrate Form 1 an absolute threshold of 0.006 with a sensitivity of 75 was utilized during peak picking. The peak position has been rounded to the nearest whole number using standard practice (0.5 rounds up, 0.4 rounds down). Peaks with normalized peak intensity between (1-0.75), (0.74-0.30), (0.29-0) were labeled as strong, medium, and weak, respectively.

The characteristic peaks for free base hemihydrate Form 1 were chosen based on intensity, as well as peak position. Comparison to free base hemihydrate Form 1, with placebo and active blends (6 and 15% DL) were conducted to ensure the uniqueness of free bae hemihydrate Form 1.

A FT-Raman spectrum peak list and normalized intensity data for the compound of Example 2, free base hemihydrate Form 1 (cm⁻¹) is provided in Table 4 below:

**Table 4**

| **Peak Position (cm⁻¹)** ± **0.2 cm⁻¹** | **Normalized Intensity** | **Classification** | **Peak Position (cm⁻¹)** ± **0.2 cm⁻¹** | **Normalized Intensity** | **Classification** |
|---|---|---|---|---|---|
| 210 | 0.33 | m | 1231 | 0.38 | m |
| 227 | 0.40 | m | 1260 | 0.37 | m |
| 246 | 0.25 | w | 1267 | 0.32 | m |
| 281 | 0.45 | m | 1286 | 0.15 | w |
| 320 | 0.26 | w | 1305 | 0.14 | w |
| 377 | 0.14 | w | 1322 | 0.32 | m |
| 402 | 0.25 | w | 1362 | 0.23 | w |
| 421 | 0.13 | w | 1379 | 0.10 | w |
| 432 | 0.13 | w | 1431 | 0.24 | w |
| 448 | 0.13 | w | 1435 | 0.24 | w |
| 486 | 0.32 | m | 1462 | 0.58 | m |
| 495 | 0.28 | w | 1529 | 1.00 | s |
| 512 | 0.19 | w | 1573 | 0.21 | w |
| 543 | 0.13 | w | 1587 | 0.23 | w |
| 555 | 0.21 | w | 1609 | 0.26 | w |
| 607 | 0.69 | m | 1904 | 0.02 | w |
| 649 | 0.22 | w | 2166 | 0.04 | w |
| 654 | 0.20 | w | 2379 | 0.02 | w |
| 719 | 0.27 | w | 2517 | 0.02 | w |
| 727 | 0.16 | w | 2701 | 0.02 | w |
| 747 | 0.15 | w | 2749 | 0.03 | w |
| 780 | 0.52 | m | 2843 | 0.26 | w |
| 803 | 0.29 | w | 2859 | 0.28 | w |
| 868 | 0.04 | w | 2881 | 0.19 | w |
| 896 | 0.06 | w | 2900 | 0.23 | w |
| 919 | 0.16 | w | 2925 | 0.45 | m |
| 931 | 0.19 | w | 2986 | 0.11 | w |
| 948 | 0.09 | w | 3016 | 0.41 | m |
| 967 | 0.13 | w | 3045 | 0.35 | m |
| 1022 | 0.62 | m | 3077 | 0.28 | w |
| 1051 | 0.52 | m | 3172 | 0.08 | w |
| 1084 | 0.36 | m | 3280 | 0.14 | w |
| 1109 | 0.18 | w | 3296 | 0.12 | w |
| 1134 | 0.75 | s | 3334 | 0.09 | w |
| 1156 | 0.43 | m | 3456 | 0.01 | w |
| 1202 | 0.23 | w | 3512 | 0.02 | w |
| 1210 | 0.30 | m | | | |

The characteristic FT-Raman peak list and normalized intensity data for the compound of Example 2, free base hemihydrate Form 1 (cm⁻¹) is provided in Table 5 below:

**Table 5**

| **Peak Position (cm⁻¹)** | **Normalized Intensity** | **Classification** |
|---|---|---|
| 607 | 0.69 | m |
| 1462 | 0.58 | m |
| 1051 | 0.52 | m |
| 1573 | 0.21 | w |

### Example 3

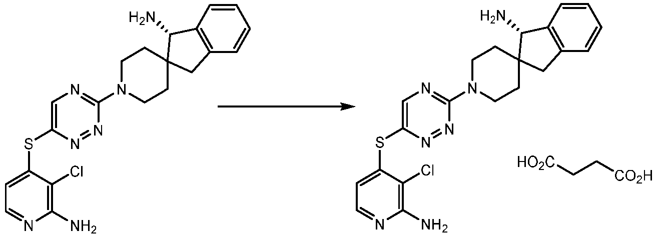

### Crystalline anhydrous (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1

Crystalline (*S*)-1'-(6-((2-amino-3-chloropyridin-4-yl)thio)-1,2,4-triazin-3-yl)-1,3-dihydrospiro[indene-2,4'-piperidin]-1-amine free base hemihydrate (Form 1) (Example 2; 8.99 Kg) was suspended in MeOH (500 L) at about 55 to 65 °C. Succinic acid (2.52 Kg) dissolved in MeOH (17.8 Kg) was added to the suspension. The solution was stirred at about 55 to 65 °C for about 2 to10 hours, and the solid appeared in the solution. The solution was cooled to about 40 to 50 °C. Methyl tert-butyl ether ("MTBE", 33.3 Kg) was added dropwise at about 40 to 50 °C. After the addition, the mixture was cooled to about 2 to 15 °C and stirred for another 2 hours at about 2 to 15 °C. The slurry was wet milled about 25 to 90 minutes at about 2 to 15 °C. The slurry was filtered and washed with MTBE (40 Kg) and dried to provide crystalline anhydrous (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 (9.4 Kg, 81% yield, 99.6% purity).

Powder X-ray diffraction analysis was conducted using a Bruker AXS D8 Endeavor diffractometer equipped with a Copper (Cu) radiation source. The divergence slit was set at 15 mm continuous illumination. Diffracted radiation was detected by a PSD-Lynx Eye detector, with the detector PSD opening set at 2.99 degrees. The X-ray tube voltage and amperage were set to 40 kV and 40 mA respectively. Data was collected in the Theta-Theta goniometer at the Cu wavelength (CuK_{α̅} = 1.5418 Å) from 3.0 to 40.0 degrees 2-Theta using a step size of 0.00998 degrees and a step time of 1.0 second. The antiscatter screen was set to a fixed distance of 3.0 mm. Samples were rotated at 15/min during collection. Samples were prepared by placing them in a silicon low background sample holder and rotated during collection. Data were collected using Bruker DIFFRAC Plus software and analysis was performed by EVA diffract plus software. The PXRD data file was not processed prior to peak searching. Using the peak search algorithm in the EVA software, peaks selected with a threshold value of 1 were used to make preliminary peak assignments. To ensure validity, adjustments were manually made; the output of automated assignments was visually checked, and peak positions were adjusted to the peak maximum. Peaks with relative intensity of ≥ 3% were generally chosen. Typically, the peaks which were not resolved or were consistent with noise were not selected. A typical error associated with the peak position from PXRD stated in USP up to +/- 0.2° 2-Theta (USP-941).

The PXRD pattern of Example 3, Form 1 anhydrous succinate salt, is shown in FIG. 1. A PXRD peak list and relative intensity data for the compound of Example 3, Form 1 anhydrous succinate salt (2-Theta °) is provided in Table 6 below:

**Table 6**

| **Angle (2 theta** °) ± **0.2 °2**θ | **Relative Intensity** (%) | **Angle (2 theta** °) ± **0.2 °2**θ | **Relative Intensity** (%) |
|---|---|---|---|
| 4.5 | 85.5 | 24.4 | 27.4 |
| 8.4 | 18.3 | 25.6 | 3.0 |
| 9.0 | 42.3 | 26.0 | 12.1 |
| 9.3 | 44.1 | 26.2 | 12.1 |
| 12.1 | 9.3 | 26.9 | 10.7 |
| 13.9 | 4.2 | 28.0 | 48.4 |
| 15.7 | 20.6 | 28.3 | 5.7 |
| 16.5 | 16.9 | 28.7 | 7.6 |
| 16.6 | 49.4 | 29.1 | 4.1 |
| 17.1 | 10.4 | 29.9 | 31.7 |
| 17.6 | 4.8 | 30.5 | 9.0 |
| 17.8 | 22.3 | 31.7 | 9.2 |
| 18.0 | 6.2 | 31.8 | 8.3 |
| 18.8 | 41.2 | 32.8 | 6.3 |
| 19.7 | 100.0 | 33.4 | 5.3 |
| 19.9 | 14.1 | 33.8 | 8.9 |
| 21.1 | 37.5 | 35.0 | 10.2 |
| 21.3 | 50.3 | 35.9 | 7.6 |
| 21.7 | 21.4 | 36.1 | 9.5 |
| 21.8 | 32.6 | 36.4 | 4.2 |
| 22.8 | 64.5 | 36.8 | 7.0 |
| 23.0 | 10.3 | 37.1 | 3.7 |
| 23.5 | 29.4 | 38.0 | 8.5 |
| 24.0 | 3.4 | 38.7 | 4.9 |
| 24.3 | 75.0 | 39.3 | 4.7 |

The characteristic PXRD peak list and relative intensity data for the compound of Example 3, Form 1 anhydrous succinate salt (2-Theta °) is provided in Table 7 below:

**Table 7**

| **Angle (° 2-Theta)** | **Relative Intensity** (%) |
|---|---|
| 19.7 | 100.0 |
| 24.3 | 75.0 |
| 9.3 | 44.1 |
| 16.6 | 49.4 |
| 9.0 | 42.3 |
| 28.0 | 48.4 |
| 18.8 | 41.2 |
| 15.7 | 20.6 |
| 12.1 | 9.3 |

Solid-state NMR (ssNMR) analysis was conducted on a CPMAS probe positioned into a Bruker-BioSpin Avance III 500 MHz (1H frequency) NMR spectrometer. Material was packed into a 4 mm ZrO₂ rotor. A magic angle spinning rate of 14.0 kHz was used. Spectra were collected at ambient temperature (temperature uncontrolled).

¹³C ssNMR spectrum were collected using a proton decoupled cross-polarization magic angle spinning (CPMAS) experiment. A phase modulated proton decoupling field of 80-100 kHz was applied during spectral acquisition. The cross-polarization contact time was set to 2 ms and the recycle delay to 11.4 seconds (hemihydrate crystalline form of (*S*)-1'-(6-((2-amino-3-chloropyridin-4-yl)thio)-1,2,4-triazin-3-yl)-1,3-dihydrospiro[indene-2,4'-piperidin]-1-amine free base (Form 1)) or 10.5 seconds (crystalline anhydrous (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1). The number of scans was adjusted to obtain an adequate signal to noise ratio. The ¹³C chemical shift scale was referenced using a ¹³C CPMAS experiment on an external standard of crystalline adamantane, setting its up-field resonance to 29.5 ppm.

Automatic peak picking was performed using Bruker-BioSpin TopSpin version 3.6 software. Generally, a threshold value of 5% relative intensity was used for preliminary peak selection. The output of the automated peak picking was visually checked to ensure validity and adjustments were manually made if necessary. Although specific solid-state NMR peak values are reported herein, there does exist a range for these peak values due to differences in instruments, samples, and sample preparation. This is common practice in the art of solid-state NMR because of the variation inherent in peak positions. A typical variability for a ¹³C chemical shift x-axis value is on the order of plus or minus 0.2 ppm for a crystalline solid. The solid-state NMR peak heights reported herein are relative intensities. Solid-state NMR intensities can vary depending on the actual setup of the CPMAS experimental parameters and the thermal history of the sample.

The ¹³C ssNMR spectrum of Example 3, Form 1 anhydrous succinate salt, is shown in FIG. 2. The peaks marked by hash marks are spinning sidebands. A ssNMR peak list and relative intensity data for the compound of Example 3, Form 1 anhydrous succinate salt is provided in Table 8 below:

**Table 8**

| **¹³C Chemical Shift (ppm)** | **Relative Intensity** (%) | **¹³C Chemical Shift (ppm)** | **Relative Intensity** (%) |
|---|---|---|---|
| 27.1 | 52 | 127.5 | 55 |
| 32.7 | 49 | 128.7 | 61 |
| 33.3 | 63 | 139.7 | 49 |
| 34.7 | 54 | 141.7 | 54 |
| 38.0 | 62 | 146.5 | 36 |
| 40.5 | 91 | 148.0 | 42 |
| 46.4 | 66 | 155.1 | 37 |
| 63.2 | 45 | 156.6 | 32 |
| 114.8 | 32 | 158.7 | 31 |
| 124.5 | 37 | 179.0 | 100 |
| 126.5 | 49 | | |

The characteristic ssNMR peak list data for the compound of Example 3, Form 1 anhydrous succinate salt is provided in Table 9 below:

**Table 9**

| **¹³C Chemical Shift (ppm)** | **Priority** |
|---|---|
| 179.0 | 1 |
| 46.4 | 2 |
| 38.0 | 3 |
| 141.7 | 4 |
| 27.1 | 5 |

Raman spectra were collected using a Thermo Scientific iS50 FT-Raman accessory attached to the FT-IR bench. A CaF2 beam splitter is utilized in the FT-Raman configuration. The spectrometer is equipped with a 1064 nm diode laser and a room temperature InGaAs detector. Prior to data acquisition, instrument performance and calibration verifications were conducted using polystyrene. Samples were analyzed in glass NMR tubes, as tablets or in a suitable sample holder held static during data collection. The spectra were collected using between 0.1 and 0.5 W of laser power and 512 co-added scans. The collection range was 3700-100 cm⁻¹. The API spectra were recorded using 2 cm⁻¹ resolution, and Happ-Genzel apodization was utilized for all of the spectra. Multiple spectra were recorded, and the reported spectrum is representative of two spots.

The intensity scale was normalized to 1 prior to peak picking. Peaks were manually identified using the Thermo Nicolet Omnic 9.7.46 software. Peak position was picked at the peak maximum, and peaks were only identified as such, if there was a slope on each side; shoulders on peaks were not included. For neat crystalline anhydrous (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 an absolute threshold of 0.06 with a sensitivity of 75 was utilized during peak picking. The peak position has been rounded to the nearest whole number using standard practice (0.5 rounds up, 0.4 rounds down). Peaks with normalized peak intensity between (1-0.75), (0.74-0.30), (0.29-0) were labeled as strong, medium and weak, respectively. The relative peak intensity values are also illustrated in this report.

The characteristic peaks for crystalline anhydrous (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 were chosen based on intensity, as well as peak position. Comparison to crystalline anhydrous (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1, with placebo and active blends (6 and 15% DL) were conducted to ensure the uniqueness of crystalline anhydrous (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1.

The full Raman spectrum of Example 3, Form 1 anhydrous succinate salt, is shown in FIG. 3. The full Raman peak list of Example 3, Form 1 anhydrous succinate salt is provided in Table 10 below:

**Table 10**

| **Peak Position (cm⁻¹)** | **Normalized Intensity** | **Classification** | **Peak Position (cm⁻¹)** | **Normalized Intensity** | **Classification** |
|---|---|---|---|---|---|
| 213 | 0.57 | m | 1217 | 0.64 | m |
| 229 | 0.38 | m | 1230 | 0.50 | m |
| 242 | 0.34 | m | 1256 | 0.41 | m |
| 268 | 0.36 | m | 1270 | 0.50 | m |
| 284 | 0.58 | m | 1297 | 0.20 | w |
| 315 | 0.36 | m | 1305 | 0.23 | w |
| 323 | 0.33 | m | 1316 | 0.25 | w |
| 374 | 0.20 | w | 1334 | 0.55 | m |
| 411 | 0.38 | m | 1353 | 0.22 | w |
| 448 | 0.18 | w | 1374 | 0.53 | m |
| 478 | 0.37 | m | 1385 | 0.21 | w |
| 494 | 0.37 | m | 1421 | 0.22 | w |
| 500 | 0.43 | m | 1436 | 0.57 | m |
| 521 | 0.30 | w | 1448 | 0.22 | w |
| 552 | 0.13 | w | 1468 | 0.80 | s |
| 600 | 0.37 | m | 1493 | 0.12 | w |
| 615 | 0.55 | m | 1530 | 0.83 | s |
| 621 | 0.54 | m | 1555 | 0.54 | m |
| 642 | 0.23 | w | 1589 | 0.41 | m |
| 652 | 0.34 | m | 1607 | 0.21 | w |
| 664 | 0.14 | w | 1690 | 0.12 | w |
| 717 | 0.33 | m | 1938 | 0.05 | w |
| 726 | 0.43 | m | 2158 | 0.06 | w |
| 752 | 0.17 | w | 2204 | 0.05 | w |
| 762 | 0.34 | m | 2373 | 0.06 | w |
| 782 | 0.68 | m | 2459 | 0.06 | w |
| 800 | 0.74 | m | 2529 | 0.07 | w |
| 817 | 0.16 | w | 2667 | 0.07 | w |
| 847 | 0.12 | w | 2739 | 0.08 | w |
| 873 | 0.10 | w | 2848 | 0.31 | m |
| 903 | 0.21 | w | 2902 | 0.75 | s |
| 916 | 0.29 | w | 2920 | 0.72 | m |
| 930 | 0.40 | m | 2943 | 1.00 | s |
| 941 | 0.26 | w | 2964 | 0.41 | m |
| 965 | 0.16 | w | 2986 | 0.18 | w |
| 1026 | 0.83 | s | 3018 | 0.45 | m |
| 1041 | 0.90 | s | 3047 | 0.49 | m |
| 1080 | 0.48 | m | 3059 | 0.58 | m |
| 1106 | 0.34 | m | 3079 | 0.53 | m |
| 1143 | 0.59 | m | 3175 | 0.06 | w |
| 1157 | 0.34 | m | 3299 | 0.04 | w |
| 1169 | 0.63 | m | 3460 | 0.03 | w |
| 1189 | 0.25 | w | 3513 | 0.02 | w |
| 1208 | 0.46 | m | | | |

| | | | | | |
|---|---|---|---|---|---|
| w = weak, peak intensity > 0.30; m = medium, peak intensity from 0.31 to 0.74; s = strong, peak intensity < 0.74 | | | | | |

The characteristic Raman peak list data for the compound of Example 3, Form 1 anhydrous succinate salt is provided in Table 11 below:

**Table 11**

| **Peak Position (cm⁻¹)** | **Normalized Intensity** | **Classification** |
|---|---|---|
| 1041 | 0.90 | s |
| 1217 | 0.64 | m |
| 1555 | 0.54 | m |
| 1026 | 0.83 | s |

### Example 4

### Crystalline (S)-1'-(6-((2-Amino-3-chloropyridin-4-yl)thio)-1,2,4-triazin-3-yl)-1,3-dihydrospiro[indene-2,4'-piperidin]-1-amine phosphate (Pattern 1)

(*S*)-1'-(6-((2-Amino-3-chloropyridin-4-yl)thio)-1,2,4-triazin-3-yl)-1,3-dihydrospiro[indene-2,4'-piperidin]-1-amine freebase (200 mg) was weighed into a 20 mL glass scintillation vial. 90% Ethanol/10% water (5 mL; %v/v) was added to the scintillation vial to form a slurry. Phosphoric acid (65.2 µL; 2.1 equiv) was added neat to the slurry. The slurry was then temperature cycled between ambient and 40 °C (4-hour cycles) with agitation for 18 hours. A sub-sample was isolated by centrifuge filtration. XPRD analysis showed phosphate Pattern 1. The remaining slurry was isolated using a Buchner funnel containing Whatmann grade 1 filter paper. The isolated solid was then dried at 40 °C under vacuum for about 24 hours.

### Example 5

### Crystalline (S)-1'-(6-((2-Amino-3-chloropyridin-4-yl)thio)-1,2,4-triazin-3-yl)-1,3-dihydrospiro[indene-2,4'-piperidin]-1-amine phosphate (Pattern 2)

(*S*)-1'-(6-((2-Amino-3-chloropyridin-4-yl)thio)-1,2,4-triazin-3-yl)-1,3-dihydrospiro[indene-2,4'-piperidin]-1-amine freebase (200 mg) was weighed into a 20 mL glass scintillation vial. 90% Ethanol/10% water (5 mL; %v/v) was added to the scintillation vial to form a slurry. Phosphoric acid (32.6 µL; 1.05 equiv) was added neat to the slurry. The slurry was then temperature cycled between ambient and 40 °C (4-hour cycles) with agitation for 18 hours. Solvent was removed using a rotary evaporator. 90% Methylethyl ketone / 10% water (%v/v) was added to the dried solid to form a slurry. The material was isolated using a Buchner funnel containing Whatmann grade 1 filter paper. Isolated solid was dried at 40 °C under vacuum for about 24 hours. This procedure produces a mixture of Pattern 1 and Pattern 2 phosphate salts.

### Example 6

### Crystalline (S)-1'-(6-((2-Amino-3-chloropyridin-4-yl)thio)-1,2,4-triazin-3-yl)-1,3-dihydrospiro[indene-2,4'-piperidin]-1-amine fumarate

(*S*)-1'-(6-((2-Amino-3-chloropyridin-4-yl)thio)-1,2,4-triazin-3-yl)-1,3-dihydrospiro[indene-2,4'-piperidin]-1-amine freebase (200 mg) was weighed into a 20 mL glass scintillation vial. Acetone (5 mL) was added to the scintillation vial to form a slurry. Fumaric acid (55.96 mg; 1.05 equivalent) was added neat to the slurry. The slurry was then temperature cycled between ambient and 40 °C (4-hour cycles) with agitation for 18 hours. Centrifugation filtration used for isolation.

### Example 7

### Crystalline (S)-1'-(6-((2-Amino-3-chloropyridin-4-yl)thio)-1,2,4-triazin-3-yl)-1,3-dihydrospiro[indene-2,4'-piperidin]-1-amine tartrate

(*S*)-1'-(6-((2-Amino-3-chloropyridin-4-yl)thio)-1,2,4-triazin-3-yl)-1,3-dihydrospiro[indene-2,4'-piperidin]-1-amine freebase (200 mg) was weighed into a 20 mL glass scintillation vial. 90% Ethanol/ 10% water (5 mL; %v/v) was added to the scintillation vial to form a slurry. L-Tartatic acid (72.36 mg; 1.05 equivalent) was added neat to the slurry. The slurry was then temperature cycled between ambient and 40 °C (4-hour cycles) with agitation for 18 hours. Centrifugation filtration used for isolation.

### Example 8

### Crystalline (S)-1'-(6-((2-Amino-3-chloropyridin-4-yl)thio)-1,2,4-triazin-3-yl)-1,3-dihydrospiro[indene-2,4'-piperidin]-1-amine hydrochloride

(*S*)-1'-(6-((2-Amino-3-chloropyridin-4-yl)thio)-1,2,4-triazin-3-yl)-1,3-dihydrospiro[indene-2,4'-piperidin]-1-amine freebase (40 mg) was weighed into an HPLC vial. 90% 2-Propanol/ 10% water (1 mL; %v/v) was added to the vial to form a slurry. Hydrochloric acid (16.0 µL; 2.1 equiv) was added neat to the slurry. The slurry was then temperature cycled between ambient and 40 °C (4-hour cycles) with agitation for 96 hours. Centrifugation filtration used for isolation

### Example 9

### Comparative analysis between salt forms of (S)-1'-(6-((2-amino-3-chloropyridin-4-yl)thio)-1,2,4-triazin-3-yl)-1,3-dihydrospiro[indene-2,4'-piperidin]-1-amine

### Solubility

The solubility (Tables 12 and 13) was determined by adding 3 mg of compound (Example 2 or 3) to 500 µL each of the appropriate buffer (pH 2 HCl, pH 4 Acetate, and pH 6 phosphate). Upon addition of the buffer solutions, the samples were vortexed. The samples were rotated at 40 °C for 8 hours, 15 °C for 5 hours, and 25 °C for 12 hours in an Envirogenie thermomixer. After the thermomixing cycle, the slurries were isolated by centrifugation filtration and the isolated mother liquors were analyzed by HPLC.

HPLC method (Tables 12 and 13): An Agilent 1290 UPLC instrument was used with a Waters XSelect HSS T3 Column (100 angstrom, 4.6 x 150 mm, 5 µm). The column temperature was set to 20 °C, the UV wavelength set to 254 nm, the injection volume was 10 µL, with a flow rate of 1.0 mL/min. 10 mM Ammonium acetate adjusted to pH 4.8 was used as a mobile phase A, and acetonitrile was used as mobile phase B. The gradient method was set up to achieve 15% mobile phase B at 0 minutes, 95% mobile phase B at 17 minutes - held until 22 minutes, 15% mobile phase B at at 22.1 minutes - held until 28 minutes.

The solubility (Table 14) was determined by adding 20 mg of salts (Examples 3 to 8) into 3 HPLC vials, to each vial, a magnetic stirrer bar was added along with 1 mL of the appropriate buffer system (pH 1.2 HCl, pH 4.5 sodium acetate, or pH 6.8 phosphate). The samples were agitated at 37 °C for 18 hours. After 24 hours, the slurries were isolated by centrifugation filtration and isolated mother liquors were analyzed by HPLC.

HPLC method (Table 14): A Dionex Ultimate 3000 HPLC instrument was used with a Waters XSelect HSS T3 Column (100 angstrom, 4.6 x 150 mm, 5 µm). The column temperature was set to 20 °C, the UV wavelength set to 254 nm, the injection volume was 10 µL, with a flow rate of 1.0 mL/min. 10 mM ammonium acetate adjusted to pH 4.8 was used as a mobile phase A, and acetonitrile was used as mobile phase B. The gradient method was set up to achieve 15% mobile phase B at 0 minutes, 95% mobile phase B at 17 minutes - held until 22 minutes, 15% mobile phase B at at 22.1 minutes - held until 28 minutes.

### Crystallinity

Polarized light microscopy ("PLM") (Table 12) was conducted with a Leitz Orthoplan optical microscope. Samples were dispersed in immersion oil (Type A) on a flat glass substrate with a glass cover plate. Samples were viewed under cross-polarized light with a 530 nm wave plate.

Powder X-ray diffraction ("PXRD") (Table 12) analysis was conducted using a Bruker AXS D8 Endeavor diffractometer equipped with a Cu radiation source. The divergence slit was set at 10 mm continuous illumination. Diffracted radiation was detected by a PSD-Lynx Eye detector, with the detector PSD opening set at 2.99 degrees. The X-ray tube voltage and amperage were set to 40 kV and 40 mA respectively. Data was collected in the Theta-Theta goniometer at the Cu wavelength from 3.0 to 40.0 degrees 2-Theta using a step size of 0.02 degrees and a step time of 0.3 second. The antiscatter screen was set to a fixed distance of 1.5 mm. Samples were rotated at 15/minute during collection. Samples were prepared by placing them in a silicon low background sample holder and rotated during collection. Data were collected using Bruker DIFFRAC Plus software and analysis was performed by EVA diffract plus software. A small divot sample holder was used.

PLM (Table 13) was conducted using less than 1 mg of API material added to a microscope slide, one drop of high viscosity immersion oil was used, and a cover slip applied. The slide was then observed under cross polarized light with a 530 nm wave plate on an Olympus BX53 Polarized Light Microscope. The sample image was collected with an integrated Olympus camera, and the photo was taken using the Olympus Stream Software.

PXRD (Table 13): Measurement parameters: The Rigaku MiniFlex 6G Diffractometer used is equipped with a Cu radiation source. Diffracted radiation was detected by a D/teX Ultra2 detector. The X-ray tube voltage and amperages were set to 40 kV and 15 mA respectively. Data was collected in the Miniflex goniometer at the Cu wavelength from 3.0 to 45.0 ° 2-theta using a step size of 0.01 ° and a step speed of 3.00 °/minute. The incident slit box was set to 1.25 ° and the length limiting slit was set at 10 mm. The sample was rotated at 10 RPM during collection. The data was analyzed using Rigaku software SmartLab Studio II. Sample Preparation: The sample was prepared by placement into a silicon low background sample holder (2 mm x 0.5 mm well). The sample was confirmed to be level and packed prior to measurement. Sample was analyzed using the instrument parameters above.

PLM (Table 14): The presence of crystallinity was determined using an Olmypus BX50 microscope, equipped with cross-polarizing lenses and a Motic camera. Images were captured using Motic Images Plus 2.0. All images were recorded using the 20x objective.

PXRD (Table 14) analysis was carried out on a PANalytical X'pert pro with PIXcel detector (128 channeles), scanning the samples between 3 and 35 °2-theta. The material was gently ground to release any agglomerates and loaded onto a multi-well plate with Kapton or Mylar polymer film to support the sample. The multi-well plate was then placed into the diffractometer and analyzed using Cu K radiation (α1 λ = 1.54060 Å; α2 = 1.54443 Å; β = 1.39225 Å; α1 : α2 ratio = 0.5) running in transmission mode (step size 0.0130 °2-Theta, step time 18.87s) using 40 kV/50 mA generator settings. Data were visualized and images generated using the HighScore Plus 4.7 desktop application (PANalytical, 2017).

### Stability

Sample Preparation (Table 12): Crystalline (*S*)-1'-(6-((2-amino-3-chloropyridin-4-yl)thio)-1,2,4-triazin-3-yl)-1,3-dihydrospiro[indene-2,4'-piperidin]-1-amine free base hemihydrate (Form 1) - Control: Compound (15.46 mg) was weighed into a 50 mL amber volumetric flask. Methanol (approximately 35 mL) was added to the flask, sonicated 4 minutes with intermittent swirling, equilibrated to room temperature, brought to volume with methanol and mixed well with repeated inversions of the flask. Crystalline (*S*)-1'-(6-((2-amino-3-chloropyridin-4-yl)thio)-1,2,4-triazin-3-yl)-1,3-dihydrospiro[indene-2,4'-piperidin]-1-amine free base hemihydrate (Form 1) - 70°C/5% relative humidity ("RH")/1week: Compound (14.83 mg) was weighed into a 50 mL amber volumetric flask. Methanol (approximately 35 mL) was added to the flask, sonicated 4 minutes with intermittent swirling, equilibrated to room temperature, brought to volume with methanol and mixed well with repeated inversions of the flask. Crystalline (*S*)-1'-(6-((2-amino-3-chloropyridin-4-yl)thio)-1,2,4-triazin-3-yl)-1,3-dihydrospiro[indene-2,4'-piperidin]-1-amine free base hemihydrate (Form 1) - 70°C/75%RH/1week: Compound (14.99 mg) was weighed into a 50 mL amber volumetric flask. Methanol (approximately 35 mL) was added to the flask, sonicated 4 minutes with intermittent swirling, equilibrated to room temperature, brought to volume with methanol and mixed well with repeated inversions to the flask.

Chemical stability (HPLC) (Table 12): A Waters Acquity H-Class instrument was used with a Waters XSelect HSS T3 Column (100 angstrom, 4.6 x 150 mm, 5 µm). The column temperature was set to 20 °C, the UV wavelength set to 254 nm, the injection volume was 10 µL, with a flow rate of 1.0 mL/min. 10 mM ammonium acetate adjusted to pH 4.8 was used as a mobile phase A, and acetonitrile was used as mobile phase B. The gradient method was set up to achieve 15% mobile phase B at 0 minutes, 95% mobile phase B at 17 minutes - held until 22 minutes, 15% mobile phase.

Physical Stability (PXRD) (Table 12): Powder X-ray diffraction analysis was conducted using a Bruker AXS D8 Endeavor diffractometer equipped with a Cu radiation source. The divergence slit was set at 10 mm continuous illumination. Diffracted radiation was detected by a PSD-Lynx Eye detector, with the detector PSD opening set at 2.99 degrees. The X-ray tube voltage and amperage were set to 40 kV and 40 mA respectively. Data was collected in the Theta-Theta goniometer at the Cu wavelength from 3.0 to 40.0 degrees 2-Theta using a step size of 0.02 degrees and a step time of 0.3 second. The antiscatter screen was set to a fixed distance of 1.5 mm. Samples were rotated at 15/min during collection. Samples were prepared by placing them in a silicon low background sample holder and rotated during collection. Data were collected using Bruker DIFFRAC Plus software and analysis was performed by EVA diffract plus software. A Small divot sample holder was used.

Photostability (Table 12): Sample preparation: Control - about 1 g of sample was transferred into a weighting bottle and covered with a glass cover. The sample was wrapped in aluminum foil to protect from light and placed into the photostability chamber. Photostability sample: about 1 g of sample was transferred into a weighing bottle, then placed into the photostability chamber. Storage conditions: The photostability samples were stored according to ICH Photostability Option 2: A cool white fluorescent lamp designed to produce an output similar to that specified in ISO 10977 (1993), a near UV fkuorescent lamp having a spectral distribution from 320 nm to 400 nm with a maximum energy emission between 350 nm and 370 nm; a significant portion of UV should be in both bands of 320 to 360 nm and 360 to 400 nm. The photo stability and dark control samples were exposed to the light equipment providing photo intensity if 5000 ± 500 Lx (near ultraviolet energy 0.84 W/m² ±10%) and irradiated for 12 days (samples exposed to light providing an overall illumination of not less than 1.2 million lux hours and an integrated near UV energy of not less than 200 watt hours/quare meter).

Photostability (HPLC) (Table 12): An Agilent 1100 HPLC was used with a Waters XSelect HSS T3 Column (100 angstrom, 4.6 x 150 mm, 5 µm). The column temperature was set to 20 °C, the UV wavelength set to 254 nm, the injection volume was 10 µL, with a flow rate of 1.0 mL/min. 10 mM ammonium acetate adjusted to pH 4.8 was used as a mobile phase A, and acetonitrile was used as mobile phase B. The gradient method was set up to achieve 15% mobile phase B at 0 minutes, 95% mobile phase B at 17 minutes - held until 22 minutes, 15% mobile phase.

### Sample Preparation (Table 13):

70 °C/75% RH Sample: about 20 mg of crystalline anhydrous (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 was placed in an open amber vial in the 70 °C/75% RH chamber

70 °C/5% RH Sample: about 20 mg crystalline anhydrous (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 was placed in a closed amber vial with the cap taped in a 70 °C chamber with uncontrolled humidity

Control Sample: about 20 mg crystalline anhydrous (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 was placed in a closed amber vial in the refrigerator

Each succinate salt Form 1 sample was prepared at ~0.3 mg/mL by transferring 1 mg of material to an amber vial, adding 3 mL of MeOH, and vortex mixing to dissolve.

Chemical Stability (HPLC) (Table 13): A Waters Acquity UPLC I-Class instrument was used with a Waters XSelect HSS T3 Column (100 angstrom, 4.6 x 150 mm, 5 µm). The column temperature was set to 20 °C, the UV wavelength set to 254 nm, the injection volume was 10 µL, with a flow rate of 1.0 mL/min. Ammonium acetate (10 mM) adjusted to pH 4.8 was used as a mobile phase A, and acetonitrile was used as mobile phase B. The gradient method was set up to achieve 15% mobile phase B at 0 minutes, 95% mobile phase B at 17 minutes; held until 22 minutes, 15% mobile phase.

Physical Stability (PXRD) (Table 13): The Rigaku MiniFlex 6G Diffractometer used is equipped with a Cu radiation source. Diffracted radiation was detected by a D/teX Ultra2 detector. The X-ray tube voltage and amperages were set to 40 kV and 15 mA respectively. Data was collected in the Miniflex goniometer at the Cu wavelength from 3.0 to 45.0 ° 2θ using a step width of 0.02° and a step speed of 2.00°/minute. The incident slit box was set to 1.25° and the length limiting slit was set at 10 mm. The sample was rotated at 10 RPM during collection. The data was analyzed using Rigaku software SmartLab Studio II.

The sample was prepared by placement into a silicon low background sample holder (0.2 indent plate). The sample was confirmed to be level and packed prior to measurement. The sample was not ground (by mortar and pestle, or by any other means) prior to placement in the sample holder.

Photostability (Table 13): Crystalline anhydrous (*S*)-1'-(6-((2-amino-3-chloropyridin-4-yl)thio)-1,2,4-triazin-3-yl)-1,3-dihydrospiro[indene-2,4'-piperidin]-1-amine mono succinate salt Form 1 - Photo-Deg Contro (Foil-Wrapped)l: 1.716 mg weighed into a 5 mL volumetric flask, approximately 3 mL methanol added to the flask, sonicated 3 minutes with intermittent swirling, equilibrated to room temperature, brought to volume with methanol and mixed well with repeated inversions of the flask.

2X ICH Photo-Deg: 1.569 mg weighed into a 5 mL volumetric flask, approximately 3 mL methanol added to the flask, sonicated 3 minutes with intermittent swirling, equilibrated to room temperature, brought to volume with methanol and mixed well with repeated inversions of the flask.

Chemical stability (HPLC) (Table 13): A Waters Acquity H-Class instrument was used with a Waters XSelect HSS T3 Column (100 angstrom, 4.6 x 150 mm, 5 µm). The column temperature was set to 20 °C, the UV wavelength set to 254 nm, the injection volume was 10 µL, with a flow rate of 1.0 mL/min. Ammonium acetate (10 mM) adjusted to pH 4.8 was used as a mobile phase A, and acetonitrile was used as mobile phase B. The gradient method was set up to achieve 15% mobile phase B at 0 minutes, 95% mobile phase B at 17 minutes; held until 22 minutes, 15% mobile phase.

Physical (PXRD) (Table 13): Powder X-ray diffraction analysis was conducted using a Bruker AXS D8 Endeavor diffractometer equipped with a Cu radiation source. The divergence slit was set at 10 mm continuous illumination. Diffracted radiation was detected by a PSD-Lynx Eye detector, with the detector PSD opening set at 2.99 degrees. The X-ray tube voltage and amperage were set to 40 kV and 40 mA respectively. Data was collected in the Theta-Theta goniometer at the Cu wavelength from 3.0 to 40.0 degrees 2-Theta using a step size of 0.02 degrees and a step time of 0.3 second. The antiscatter screen was set to a fixed distance of 1.5 mm. Samples were rotated at 15/min during collection. Samples were prepared by placing them in a silicon low background sample holder and rotated during collected using Bruker DIFFRAC Plus software and analysis was performed by EVA diffract plus software.

Thermogravimetric Analysis ("TGA") (Table 12): Thermogravimetric analysis was conducted using a Discovery TGA (TA instruments) thermogravimetric analyzer. Samples of approximately 10 mg were weighed into aluminum pans and heated from ambient to 300 °C at 10 °C/minute heating rate under nitrogen purge (10 mL/min for both sample chamber and balance).

Differential Scanning Calorimetry ("DSC") (Table 12): DSC measurements were performed with Discovery DSC (TA instruments) equipped with a refrigerated cooling accessory. All the experiments were performed in standard/Tzero aluminum pans. The cell constant was determined using indium and temperature calibration was performed using indium and tin as standards. All the measurements were done under continuous dry nitrogen purge (50 mL/min). Approximately 1-5 mg of solid sample was weighed into a Tzero aluminum pan, sealed nonhermetically and heated from 0 °C to 250 °C at 10 °C/min heating rate. The experimental data were analyzed using commercially available software (TA Universal Analysis 2000/Trios software, TA Instruments).

Dynamic Vapor Sorption ("DVS") (Table 12): The microbalance was calibrated using a 100 mg standard weight. The relative humidity sensor was calibrated at 5.0, 11.3, 32.8, 52.8, 75.3, and 84.3% RH (25°C) using saturated salt solutions, as well as 80% RH (25°C) using polyvinylpyrrolidone. 8-10 mg of the powder sample was placed in the platinum sample pan. There is no drying step with this method. The RH is first maintained at 30% RH, then decreased to 10% RH, and then 3%RH using the criteria of moving on when the weight change of the sample was < 0.001wt% in 5 min or after reaching a maximum equilibration time of 60 minutes. The RH was then progressively increased to 90% in increments of 10% followed by a decrease to a final RH of 10% in 10% RH increments. Again, the attainment of equilibrium was assumed when the weight change of the sample was < 0.001wt% in 5 min or by a maximum equilibration time of 120 minutes.

DVS (Table 13): Water sorption and desorption were analyzed with a DVS-Resolution. The microbalance is calibrated with a 100 mg standard weight on a monthly basis. About 5 mg of API was added to a translucent sample pan and placed inside chamber A of the DVS-Resolution. Percent relative humidity was held at 0% for 1 hour, and increased to 90% in 10% increments, then ramped back to 0% in 10% increments. Steps were considered complete when a dm/dt change of < 0.001% was observed with a minimum of 30 minutes or a maximum step time of 120 minutes was reached.

TGA/DSC (Table 13): A Simultaneous DSC/TGA instrument was used for the thermal analysis. Approximately 3.2 mg of the API was placed in an aluminum Tzero DSC pan was loaded into the instrument via autosampler. The sample was equilibrated to 35°C in the instrument prior to analysis and was ramped to 400°C at a ramp rate of 10°C/min. Dry nitrogen gas from the in-house supply was used.

TGA/DSC (Table 14): Approximately, 5-10 mg of material was added into a pre-tared open aluminum pan and loaded into a TA Instruments Discovery SDT 650 Auto - Simultaneous DSC and held at room temperature. The sample was then heated at a rate of 10 °C/min from 20 °C (ambient) to 400 °C during which time the change in sample weight was recorded along with the heat flow response (DSC). Nitrogen was used as the sample purge gas, at a flow rate of 200 cm3/min.

DSC (Table 14): Approximately, 1-5 mg of material was weighed into an aluminum DSC pan and sealed nonhermetically with an aluminum lid. The sample pan was then loaded into a TA Instruments Discovery DSC 2500 differential scanning calorimeter equipped with a RC90 cooler. The sample and reference were heated to 310°C at a scan rate of 10 °C/min and the resulting heat flow response monitored. The sample was re-cooled to 20°C and then reheated again to 205 °C all at 10 °C/min. Nitrogen was used as the purge gas, at a flow rate of 50 cm³/min.

DVS (Table 14): Approximately, 10-20 mg of sample was placed into a mesh vapor sorption balance pan and loaded into a DVS Intrinsic dynamic vapor sorption balance by Surface Measurement Systems. The sample was subjected to a ramping profile from 40 - 90 % relative humidity (RH) at 10 % increments, maintaining the sample at each step until a stable weight had been achieved (dm/dt 0.004 %, minimum step length 30 minutes, maximum step length 500 minutes) at 25 °C. After completion of the sorption cycle, the sample was dried using the same procedure to 0% RH and then a second sorption cycle back to 40% RH. Two cycles were performed. Theweight change during the sorption/desorption cycles were plotted, allowing for the hygroscopic nature of the sample to be determined. XRPD analysis was then carried out on any solid retained. Approximately, 10-20 mg of sample was placed into a mesh vapor sorption balance pan and loaded into a DVS Advantage dynamic vapor sorption balance by Surface Measurement Systems. The sample was subjected to a ramping profile from 40 - 90 % relative humidity (RH) at 10 % increments, maintaining the sample at each step until a stable weight had been achieved (dm/dt 0.004 %, minimum step length 30 minutes, maximum step length 500 minutes) at 25 °C. After completion of the sorption cycle, the sample was dried using the same procedure to 0 %RH and then a second sorption cycle back to 40% RH. Two cycles were performed. The weight change during the sorption/desorption cycles were plotted, allowing for the hygroscopic nature of the sample to be determined. XRPD analysis was then carried out on any solid retained.

NMR (Table 14): NMR experiments were performed on a Bruker AVIIIHD spectrometer equipped with a DCH cryoprobe operating at 500.12MHz for protons. Experiments were performed in deuterated dimethylsulfoxide ((CD₃)₂SO) and methanol (CD₃OD) and each sample was prepared to about 10 mM concentration.

Table 12 shows the properties of crystalline free base hemihydrate Form 1 of Example 2. Table 13 shows the properties of crystalline mono succinate salt Form 1 of Example 3.

**Table 12**

| **Crystalline Free Base Hemihydrate Form 1 (Example 2)** | | | | |
|---|---|---|---|---|
| **Solubility** | **Concentration (µg/mL)** | **Final pH** | **% Purity** | **Final XRD** |
| pH 3 | 2044.85 | 4.51 | 98.34 | Freebase Form 1 |
| pH 4 | 301.79 | 5.12 | 90.87 | Freebase Form 1 |
| pH 5 | 58.53 | 6.06 | 82.5 | Freebase Form 1 |
| pH 6 | 32.25 | 6.95 | 89.75 | Freebase Form 1 |
| pH 7 | 10.05 | 8.19 | 91.26 | Freebase Form 1 |
| pH 8 | 0.56 | 9.13 | 36.92 | Insufficient solids |
| pH 9 | 0.26 | 10.2 | 16.42 | Insufficient solids |
| pH 10 | 0.45 | 3.94 | 32.48 | Insufficient solids |
| Water | 0.24 | 9.19 | 15.04 | Additional peak at 8° |

| Crystallinity | | | | |
|---|---|---|---|---|
| Crystallinity XRD/PLM | Highly Crystalline | | | |
| Grinding Assessment | No change in form or crystallinity | | | |

| Stability | | | | |
|---|---|---|---|---|
| Chemical Stability (70 °C/75% RH) | | 0.09% decrease in purity | | |
| Chemical Stability (70 °C/5% RH) | | 0.25% decrease in purity | | |
| Physical Stability (70 °C/75% RH) | | No change | | |
| Physical Stability (70 °C/75% RH) | | No change | | |
| Photostability (2xICH) | | Two photodegs: RRT 1.16 ~ 0.36%, RRT 1.62 ~ 0.32% | | |

| **Thermal analysis and Hygroscopity** | | | | |
|---|---|---|---|---|
| Melt Onset (°C) | | ~147 | | |
| Dehyradtion Onset (°C) | | ~72 | | |
| Decomposition Onset (°C) | | ~240 | | |
| Weight loss | | ~2.4% by 150 °C | | |
| Hygroscopity | | Non-hygroscopic at 25 °C and exhibits less than ~0.3% weight gain upon exposure to 90% relative humidity | | |

**Table 13**

| **Crystalline Mono Succinate Salt Form 1 (Example 3)** | | | | |
|---|---|---|---|---|
| **Solubility** | **Concentration (µg/mL)** | **Final pH** | **% Purity** | **Final XRD** |
| pH 3 | 4215.04 | 4.5 | 97.76 | Succinate Form 1 |
| pH 4 | 6398.51 | 4.16 | 99.29 | Insufficient solids |
| pH 5 | 4439.44 | 5.08 | 99.11 | Succinate Form 1 |
| pH 6 | 597.17 | 5.37 | 97.34 | Freebase Form 1 |
| pH 7 | 17.11 | 6.81 | 90.52 | Insufficient solids |
| pH 8 | 2.66 | 7.78 | 64.4 | Freebase Form 1 |
| pH 9 | 0.18 | 9.01 | 13.1 | Freebase Form 1 |
| pH 10 | 0.17 | 9.82 | 10.16 | Freebase Form 1 |
| Water | 8177.63 | 5.01 | 98.66 | Succinate Form 1 |

| **Crystallinity** | | | | |
|---|---|---|---|---|
| Crystallinity XRD/PLM | Highly Crystalline | | | |
| Grinding Assessment | Reduction in crystallinity | | | |

| **Stability** | | | | |
|---|---|---|---|---|
| Chemical Stability (70 °C/75% RH) | | 0.19% decrease in purity | | |
| Chemical Stability (70 °C/5% RH) | | 0.13% decrease in purity | | |
| Physical Stability (70 °C/75% RH) | | No change | | |
| Physical Stability (70 °C/75% RH) | | No change | | |
| Photostability (2xICH) | | 0.53% decrease in purity | | |

| **Thermal analysis and Hygroscopity** | | | | |
|---|---|---|---|---|
| Melt Onset (°C) | | ~194 | | |
| Dehyradtion Onset (°C) | | N/A | | |
| Decomposition Onset (°C) | | ~180 | | |
| Weight loss | | 2% | | |
| Hygroscopity | | 0.9% weight gain, not hygroscopic | | |

Table 14 shows the comparison data of salts of (S)-1'-(6-((2-amino-3-chloropyridin-4-yl)thio)-1,2,4-triazin-3-yl)-1,3-dihydrospiro[indene-2,4'-piperidin]-1-amine.

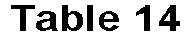

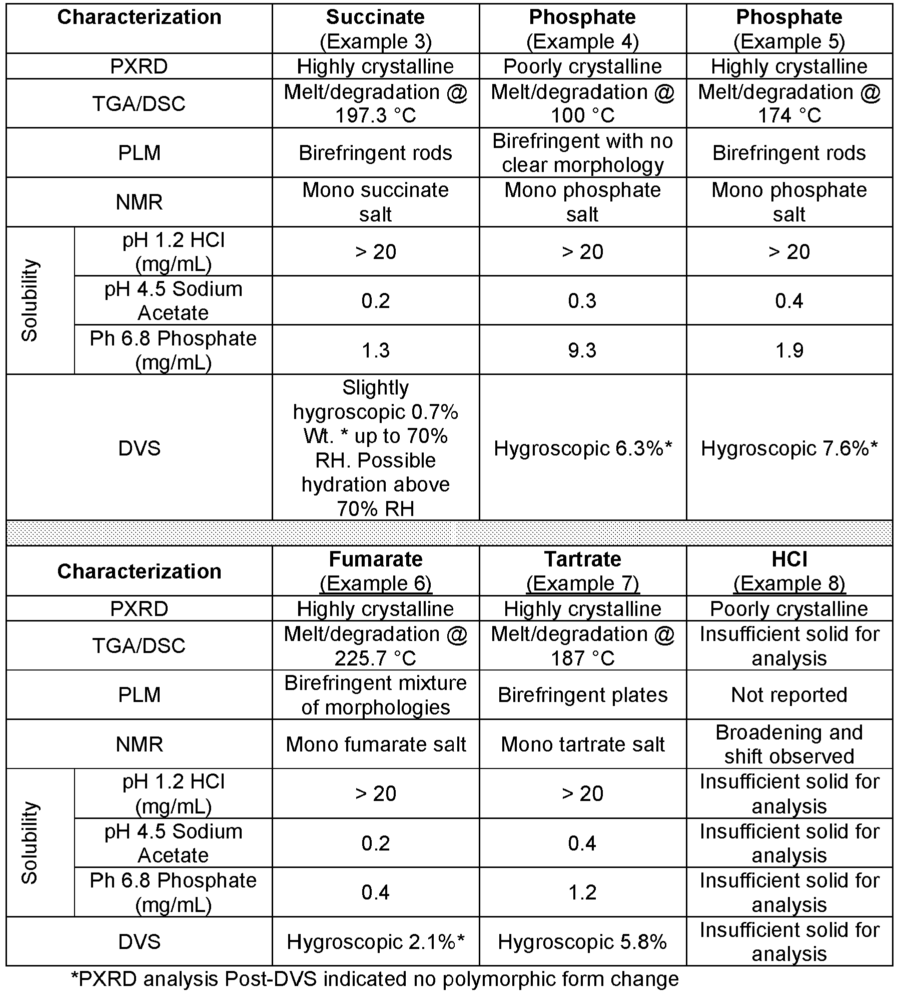

### Example 10

### Pharmaceutical formulation of crystalline anhydrous (S)-1'-(6-((2-amino-3-chloropyridin-4-yl)thio)-1,2,4-triazin-3-yl)-1,3-dihydrospiro[indene-2,4'-piperidin]-1-amine mono succinate salt Form 1

(S)-1'-(6-((2-Amino-3-chloropyridin-4-yl)thio)-1,2,4-triazin-3-yl)-1,3-dihydrospiro[indene-2,4'-piperidin]-1-amine mono succinate salt Form 1 is formulated to make 10 mg, 25 mg (common blend 50 mg/g used for 10 mg and 25 mg tablets) and 100 mg immediate release tablets. The formulation, provided in Tables 15 and 16, is composed of microcrystalline cellulose and dibasic calcium phosphate anhydrous (diluents), crospovidone (disintegrant), and sodium stearyl fumarate (lubricant). (S)-1'-(6-((2-Amino-3-chloropyridin-4-yl)thio)-1,2,4-triazin-3-yl)-1,3-dihydrospiro[indene-2,4'-piperidin]-1-amine mono succinate salt Form 1 tablet cores are manufactured using a dry granulation manufacturing.

Tablet composition for 10 mg and 25 mg tablets manufactured using common blend of 50 mg/g active is shown in Table 15 and tablet composition for 100 mg tablets is shown in Table 16.

**Table 15**

| | | | | Tablet Strength | |
|---|---|---|---|---|---|
| Ingredient | % Composition | Reference to Standards | Function | 10 mg | 25 mg |
| | | | | Unit Formula (mg/tablet) | |
| | | | | Tablet Core Composition | |
| Succinate Salt Form 1¹ | 6.34 | Pfizer | Active ingredient | 12.674 | 31.686 |
| Microcrystalline Cellulose² | 59.11 | NF/Ph | Diluent | 118.226 | 295.564 |
| | | Eur/JP | | | |
| Dibasic Calcium Phophate | 29.55 | NF/Ph | Diluent | 59.100 | 147.750 |
| | | Eur/JP | | | |
| Crospovidone | 3.00 | NF/Ph | Disintegrant | 6.000 | 15.000 |
| | | Eur/JP | | | |
| Sodium stearyl fumarate (non-bovine) | 2.00 | NF/Ph | Lubricant | 4.000 | 10.000 |
| | | Eur/JP | | | |
| Tablet Core Weight | 100.00 | | | 200.000 | 500.000 |

| | | | | | |
|---|---|---|---|---|---|
| ¹ Based upon a theoretical potency of 0.789. Quantity may be adjusted according to actual drug substance potency ² Quantity of microcrystalline cellulos may be adjusted for slight potency changes of drug substance to ensure constant table weight NF = National Formulary; Ph Eur = European Pharmacopeia; JP = Japanes Pharmacopeia | | | | | |

**Table 16**

| | | | | Tablet Strength |
|---|---|---|---|---|
| Ingredient | % Composition | Reference to Standards | Function | 100 mg |
| | | | | Unit Formula (mg/tablet) |
| | | | | Tablet Core Composition |
| Succinate Salt Form 1¹ | 14.92 | Pfizer | Active ingredient | 126.743 |
| Microcrystalline Cellulose² | 53.38 | NF/Ph | Diluent | 453.807 |
| | | Eur/JP | | |
| Dibasic Calcium Phophate | 26.70 | NF/Ph | Diluent | 226.950 |
| | | Eur/JP | | |
| Crospovidone | 3.00 | NF/Ph | Disintegrant | 25.500 |
| | | Eur/JP | | |
| Sodium stearyl fumarate (non-bovine) | 2.00 | NF/Ph | Lubricant | 17.000 |
| | | Eur/JP | | |
| Tablet Core Weight | 100.00 | | | 850.000 |

| | | | | |
|---|---|---|---|---|
| ¹ Based upon a theoretical potency of 0.789. Quantity may be adjusted according to actual drug substance potency ² Quantity of microcrystalline cellulos may be adjusted for slight potency changes of drug substance to ensure constant table weight | | | | |

(S)-1'-(6-((2-Amino-3-chloropyridin-4-yl)thio)-1,2,4-triazin-3-yl)-1,3-dihydrospiro[indene-2,4'-piperidin]-1-amine mono succinate salt Form 1 immediate release 10, 25 and 100 mg tablet cores are manufactured using a platform designed and developed for batch dry granulation of solid, oral immediate release tablets.

The diluents (microcrystalline cellulose part 1 of 2) and disintegrant are blended first, followed by addition of active ingredient and blending. The remaining microcrystalline cellulose (part 2 of 2) and the active blend (previous step) are passed through co-mill to deagglomerate any aggregates. This is followed by blending for uniformity post milling. Lubrication with sodium stearyl fumarate is performed to prepare the uniform blend. This blend is dry granulated (using roller compaction or slugging) and the slugs obtained are milled (using in-line mill or co-mill). The milled granules are blended to obtain uniform distribution of the granule particles. Lubrication of the granules is performed to obtain uniform lubricated granules. Finally, the uniform blend is delivered to the tablet press for compression of tablet cores.

## Claims

1. A crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt.

2. The crystalline form of Claim 1, **characterized by** one of the following: 1) a powder X-ray diffraction pattern comprising peaks at 2θ values of: 19.7, 24.3, 9.3 and 16.6 ± 0.2 °2θ; (2) a Raman spectrum comprising wavenumber values of: 1041 and 1217 cm⁻¹ ±2 cm⁻¹; or (3) a¹³C solid state NMR spectrum comprising a resonance value of: 179.0 ppm ± 0.2 ppm.

3. The crystalline form of Claim 1 or 2, **characterized by** two of the following: 1) a powder X-ray diffraction pattern comprising peaks at 2θ values of: 19.7, 24.3, 9.3 and 16.6 ± 0.2 °2θ; (2) a Raman spectrum comprising wavenumber values of: 1041 and 1217 cm⁻¹ ±2 cm⁻¹; or (3) a ¹³C solid state NMR spectrum comprising a resonance value of: 179.0 ppm ± 0.2 ppm.

4. The crystalline form of any one of Claims 1 to 3, **characterized by**: 1) a powder X-ray diffraction pattern comprising peaks at 2θ values of: 19.7, 24.3, 9.3 and 16.6 ± 0.2 °2θ; (2) a Raman spectrum comprising wavenumber values of: 1041 and 1217 cm⁻¹ ±2 cm⁻¹; and (3) a ¹³C solid state NMR spectrum comprising a resonance value of: 179.0 ppm ± 0.2 ppm.

5. The crystalline form of any one of Claims 1 to 4, having a powder X-ray diffraction pattern further comprising a peak at the 2θ value of: 9.0 ± 0.2 °2θ.

6. The crystalline form of any one of Claims 1 to 5, having a powder X-ray diffraction pattern further comprising a peak at the 2θ value of: 28.0 ± 0.2 °2θ.

7. The crystalline form of any one of Claims 1 to 6, having a powder X-ray diffraction pattern further comprising a peak at the 2θ value of: 18.8 ± 0.2 °2θ.

8. The crystalline form of any one of Claims 1 to 7, having a powder X-ray diffraction pattern further comprising a peak at the 2θ value of: 15.7 ± 0.2 °2θ.

9. The crystalline form of any one of Claims 1 to 8, having a powder X-ray diffraction pattern further comprising a peak at the 2θ value of: 12.1 ± 0.2 °2θ.

10. The crystalline form of any one of Claims 1 to 9, having a Raman spectrum comprising wavenumber (cm⁻¹) values of: 1026 and 1555 cm⁻¹ ± 2 cm⁻¹.

11. The crystalline form of any one of Claims 1 to 10, having a ¹³C solid state NMR spectrum comprising a resonance (ppm) value of: 46.4 ppm ± 0.2 ppm, 38.0 ppm ± 0.2 ppm, 141.7 ppm ± 0.2 ppm, or 27.1 ppm ± 0.2 ppm.

12. The crystalline form of any one of Claims 1 to 11 which is substantially pure and free of alternative forms.

13. A pharmaceutical composition comprising the crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt according to any one of Claims 1 to 12, and at least one pharmaceutically acceptable excipient.

14. The crystalline form of any one of Claims 1 to 12 for use as a medicament.

15. The crystalline anhydrous form of (S)-1'-(6-((2-amino-3-chloropyridin-4-yl)thio)-1,2,4-triazin-3-yl)-1,3-dihydrospiro[indene-2,4'-piperidin]-1-amine mono succinate salt according to any one of Claims 1 to 12 for use in the treatment of abnormal cell growth in a mammal, wherein the mammal is human.

16. The compound for use according to Claim 15, wherein the abnormal cell growth is cancer.

17. The compound for use according to Claim 16, wherein the cancer is selected from the group consisting of melanoma, juvenile myelomoncytic leukemias, neuroblastoma, Philadelphia chromosome positive chronic myeloid leukemia, Philadelphia chromosome positive acute lymphoblastic leukemias, acute myeloid leukemias, myeloproliferative neoplasms, breast cancer, lung cancer, liver cancer, colorectal cancer, esophageal cancer, gastric cancer, squamous-cell carcinoma of the head and neck, glioblastoma, anaplastic large-cell lymphoma, thyroid carcinoma, spitzoid neoplasms, non-small cell lung cancer, colon cancer, esophageal cancer, rectal cancer, juvenile myelomonocytic leukemia, breast cancer, melanoma, and pancreatic cancer ALK-positive NSCLC, ROS1-positive NSCLC, BRAF V600E mutation colorectal cancer, RAS-mutant solid tumors, NF1-mutant solid tumors, and BRAF class 3-mutant solid tumors.

18. The compound for use according to Claim 17, wherein the cancer is ALK-positive NSCLC.

19. The compound for use according to Claim 17, wherein the cancer is BRAF V600E mutation colorectal cancer.

20. The compound for use according to Claim 17, wherein the cancer is RAS-mutant solid tumors.

21. The compound for use according to Claim 17, wherein the cancer is NF1-mutant solid tumors.

22. The compound for use according to Claim 17, wherein the cancer is BRAF class 3-mutant solid tumors.

23. The compound for use according to Claim 16, wherein the cancer is a KRAS mutation cancer.

24. The compound for use according to Claim 23, wherein the KRAS mutation is selected from a KRASG12A mutation, a KRASG12C mutation, a KRASG12D mutation, a KRASG12F mutation, a KRASG12I mutation, a KRASG12L mutation, a KRASG12R mutation, a KRASG12S mutation, a KRASG12V mutation, and a KRASG12Y mutation.

25. The compound according to any one of Claims 1 to 12 for use in combination with an additional therapeutic compound.

26. The compound for use according to Claim 25, wherein the additional therapeutic is selected from the group consisting of lorlatinib, binimietinib, cetuximab, and encorafenib.

## Patentansprüche

1. Kristalline wasserfreie Form des (S)-1'-(6-((2-Amino-3-chlorpyridin-4-yl)thio)-1,2,4-triazin-3-yl)-1,3-dihydrospiro[inden-2,4'-piperidin]-1-amin-monosuccinatsalzes.

2. Kristalline Form gemäß Anspruch 1, **gekennzeichnet durch** eines der folgenden Merkmale: 1) ein Pulver-Röntgenbeugungsmuster mit Peaks bei 2θ-Werten von: 19,7, 24,3, 9,3 und 16,6 ± 0,2 °2θ; (2) ein Raman-Spektrum mit Wellenzahlwerten von: 1041 und 1217 cm⁻¹ ± 2 cm⁻¹; oder (3) ein ¹³C-Festkörper-NMR-Spektrum mit einem Resonanzwert von: 179,0 ppm ± 0,2 ppm.

3. Kristalline Form gemäß Anspruch 1 oder 2, **gekennzeichnet durch** zwei der folgenden Merkmale: 1) ein Pulver-Röntgenbeugungsmuster mit Peaks bei 2θ-Werten von: 19,7, 24,3, 9,3 und 16,6 ± 0,2 °2θ; (2) ein Raman-Spektrum mit Wellenzahlwerten von: 1041 und 1217 cm⁻¹ ± 2 cm⁻¹; oder (3) ein ¹³C-Festkörper-NMR-Spektrum mit einem Resonanzwert von: 179,0 ppm ± 0,2 ppm.

4. Kristalline Form gemäß einem der Ansprüche 1 bis 3, **gekennzeichnet durch**: 1) ein Pulver-Röntgenbeugungsmuster mit Peaks bei 2θ-Werten von: 19,7, 24,3, 9,3 und 16,6 ± 0,2 °2θ; (2) ein Raman-Spektrum mit Wellenzahlwerten von: 1041 und 1217 cm⁻¹ ±2 cm⁻¹; und (3) ein ¹³C-Festkörper-NMR-Spektrum mit einem Resonanzwert von: 179,0 ppm ± 0,2 ppm.

5. Kristalline Form gemäß einem der Ansprüche 1 bis 4, mit einem Pulver-Röntgenbeugungsmuster, das ferner einen Peak bei einem 2θ-Wert von 9,0 ± 0,2 °2θ umfasst.

6. Kristalline Form gemäß einem der Ansprüche 1 bis 5, mit einem Pulver-Röntgenbeugungsmuster, das ferner einen Peak bei einem 2θ-Wert von 28,0 ± 0,2 °2θ umfasst.

7. Kristalline Form gemäß einem der Ansprüche 1 bis 6, mit einem Pulver-Röntgenbeugungsmuster, das ferner einen Peak bei einem 2θ-Wert von 18,8 ± 0,2 °2θ umfasst.

8. Kristalline Form gemäß einem der Ansprüche 1 bis 7, mit einem Pulver-Röntgenbeugungsmuster, das ferner einen Peak bei einem 2θ-Wert von 15,7 ± 0,2 °2θ umfasst.

9. Kristalline Form gemäß einem der Ansprüche 1 bis 8, mit einem Pulver-Röntgenbeugungsmuster, das ferner einen Peak bei einem 2θ-Wert von 12,1 ± 0,2 °2θ umfasst.

10. Kristalline Form gemäß einem der Ansprüche 1 bis 9, mit einem Raman-Spektrum, das Wellenzahlwerte (cm⁻¹) von 1026 und 1555 cm⁻¹ ± 2 cm⁻¹ umfasst.

11. Kristalline Form gemäß einem der Ansprüche 1 bis 10, mit einem ¹³C-Festkörper-NMR-Spektrum mit einem Resonanzwert (ppm) von: 46,4 ppm ± 0,2 ppm, 38,0 ppm ± 0,2 ppm, 141,7 ppm ± 0,2 ppm oder 27,1 ppm ± 0,2 ppm.

12. Kristalline Form gemäß einem der Ansprüche 1 bis 11, die im Wesentlichen rein und frei von alternativen Formen ist.

13. Pharmazeutische Zusammensetzung, umfassend die kristalline wasserfreie Form des (S)-1'-(6-((2-Amino-3-chlorpyridin-4-yl)thio)-1,2,4-triazin-3-yl)-1,3-dihydrospiro[inden-2,4'-piperidin]-1-amin-monosuccinatsalzes gemäß einem der Ansprüche 1 bis 12 und mindestens einen pharmazeutisch verträglichen Hilfsstoff.

14. Kristalline Form gemäß einem der Ansprüche 1 bis 12 zur Verwendung als Arzneimittel.

15. Kristalline wasserfreie Form des (S)-1'-(6-((2-Amino-3-chlorpyridin-4-yl)thio)-1,2,4-triazin-3-yl)-1,3-dihydrospiro[inden-2,4'-piperidin]-1-amin-monosuccinatsalzes gemäß einem der Ansprüche 1 bis 12 zur Verwendung bei der Behandlung von abnormalem Zellwachstum bei einem Säugetier, wobei das Säugetier ein Mensch ist.

16. Verbindung zur Verwendung gemäß Anspruch 15, wobei das abnormale Zellwachstum Krebs ist.

17. Verbindung zur Verwendung gemäß Anspruch 16, wobei der Krebs ausgewählt ist aus der Gruppe bestehend aus Melanomen, juvenilen myelomonozytären Leukämien, Neuroblastomen, Philadelphia-Chromosom-positiven chronischen myeloischen Leukämien, Philadelphia-Chromosom-positiven akuten lymphoblastischen Leukämien, akuten myeloischen Leukämien, myeloproliferativen Neoplasien, Brustkrebs, Lungenkrebs, Leberkrebs, Darmkrebs, Speiseröhrenkrebs, Magenkrebs, Plattenepithelkarzinomen des Kopfes und Halses, Glioblastomen, anaplastischen großzelligen Lymphomen, Schilddrüsenkarzinomen, spitzoiden Neoplasien, nicht-kleinzelligem Lungenkarzinom, Kolonkarzinom, Ösophaguskarzinom, Rektumkarzinom, juveniler myelomonozytärer Leukämie, Brustkrebs, Melanom und Pankreaskarzinom, ALK-positivem NSCLC, ROS1-positivem NSCLC, BRAF-V600E-mutiertem kolorektalem Karzinom, RAS-mutierten soliden Tumoren, NF1-mutierten soliden Tumoren und BRAF-Klasse-3-mutierten soliden Tumoren.

18. Verbindung zur Verwendung gemäß Anspruch 17, wobei es sich bei dem Krebs um einen ALK-positiven NSCLC handelt.

19. Verbindung zur Verwendung gemäß Anspruch 17, wobei es sich bei dem Krebs um einen BRAF-V600E-mutierten Darmkrebs handelt.

20. Verbindung zur Verwendung gemäß Anspruch 17, wobei es sich bei dem Krebs um RAS-mutierte solide Tumore handelt.

21. Verbindung zur Verwendung gemäß Anspruch 17, wobei es sich bei dem Krebs um NF1-mutierte solide Tumore handelt.

22. Verbindung zur Verwendung gemäß Anspruch 17, wobei es sich bei dem Krebs um BRAF-Klasse-3-mutierte solide Tumore handelt.

23. Verbindung zur Verwendung gemäß Anspruch 16, wobei es sich bei dem Krebs um einen KRAS-mutierten Krebs handelt.

24. Verbindung zur Verwendung gemäß Anspruch 23, wobei die KRAS-Mutation ausgewählt ist aus einer KRASG12A-Mutation, einer KRASG12C-Mutation, einer KRASG12D-Mutation, einer KRASG12F-Mutation, einer KRASG12I-Mutation, einer KRASG12L-Mutation, einer KRASG12R-Mutation, einer KRASG12S-Mutation, einer KRASG12V-Mutation und einer KRASG12Y-Mutation.

25. Verbindung gemäß einem der Ansprüche 1 bis 12 zur Verwendung in Kombination mit einer zusätzlichen therapeutischen Verbindung.

26. Verbindung zur Verwendung gemäß Anspruch 25, wobei die zusätzliche therapeutische Verbindung ausgewählt ist aus der Gruppe bestehend aus Lorlatinib, Binimietinib, Cetuximab und Encorafenib.

## Revendications

1. Forme anhydre cristalline de sel de (S)-1'-(6-((2-amino-3-chloropyridin-4-yl)thio)-1,2,4-triazin-3-yl)-1,3-dihydrospiro[indène-2,4'-pipéridin]-1-amine monosuccinate.

2. Forme cristalline selon la revendication 1, **caractérisée par** l'un des éléments suivants : 1) un diagramme de diffraction de rayons X sur poudre comportant des pics à des valeurs 2θ de : 19,7, 24,3, 9,3 et 16,6 ± 0,2 °2θ ; (2) un spectre Raman comportant des valeurs de nombre d'onde de : 1041 et 1217 cm⁻¹ ± 2 cm⁻¹ ; ou (3) un spectre RMN à l'état solide ¹³C comportant une valeur de résonance de : 179,0 ppm ± 0,2 ppm.

3. Forme cristalline selon la revendication 1 ou 2, **caractérisée par** deux des éléments suivants : 1) un diagramme de diffraction de rayons X sur poudre comportant des pics à des valeurs 2θ de : 19,7, 24,3, 9,3 et 16,6 ± 0,2 °2θ ; (2) un spectre Raman comportant des valeurs de nombre d'onde de : 1041 et 1217 cm⁻¹ ± 2 cm⁻¹ ; ou (3) un spectre RMN à l'état solide ¹³C comportant une valeur de résonance de : 179,0 ppm ± 0,2 ppm.

4. Forme cristalline selon l'une quelconque des revendications 1 à 3, **caractérisée par** : 1) un diagramme de diffraction de rayons X sur poudre comportant des pics à des valeurs 2θ de : 19,7, 24,3, 9,3 et 16,6 ± 0,2 °2θ ; (2) un spectre Raman comportant des valeurs de nombre d'onde de : 1041 et 1217 cm⁻¹ ± 2 cm⁻¹ ; et (3) un spectre RMN à l'état solide ¹³C comportant une valeur de résonance de : 179,0 ppm ± 0,2 ppm.

5. Forme cristalline selon l'une quelconque des revendications 1 à 4, présentant un diagramme de diffraction de rayons X sur poudre comportant en outre un pic à la valeur 2θ de : 9,0 ± 0,2 °2θ.

6. Forme cristalline selon l'une quelconque des revendications 1 à 5, présentant un diagramme de diffraction de rayons X sur poudre comportant en outre un pic à la valeur 2θ de : 28,0 ± 0,2 °2θ.

7. Forme cristalline selon l'une quelconque des revendications 1 à 6, présentant un diagramme de diffraction de rayons X sur poudre comportant en outre un pic à la valeur 2θ de : 18,8 + 0,2 °2θ.

8. Forme cristalline selon l'une quelconque des revendications 1 à 7, présentant un diagramme de diffraction de rayons X sur poudre comportant en outre un pic à la valeur 2θ de : 15,7 ± 0,2 °2θ.

9. Forme cristalline selon l'une quelconque des revendications 1 à 8, présentant un diagramme de diffraction de rayons X sur poudre comportant en outre un pic à la valeur 2θ de : 12,1 ± 0,2 °2θ.

10. Forme cristalline selon l'une quelconque des revendications 1 à 9, présentant un spectre Raman comportant des valeurs de nombre d'onde (cm⁻¹) de : 1026 et 1555 cm⁻¹ ± 2 cm⁻¹.

11. Forme cristalline selon l'une quelconque des revendications 1 à 10, présentant un spectre RMN à l'état solide ¹³C comportant une valeur de résonance (ppm) de : 46,4 ppm ± 0,2 ppm, 38,0 ppm ± 0,2 ppm, 141,7 ppm ± 0,2 ppm ou 27,1 ppm ± 0,2 ppm.

12. Forme cristalline selon l'une quelconque des revendications 1 à 11 qui est sensiblement pure et exempte de formes alternatives.

13. Composition pharmaceutique comportant la forme anhydre cristalline de sel de (S)-1'-(6-((2-amino-3-chloropyridin-4-yl)thio)-1,2,4-triazin-3-yl)-1,3-dihydrospiro[indène-2,4'-pipéridin]-1-amine monosuccinate selon l'une quelconque des revendications 1 à 12, et au moins un excipient pharmaceutiquement acceptable.

14. Forme cristalline selon l'une quelconque des revendications 1 à 12 pour une utilisation comme médicament.

15. Forme anhydre cristalline de sel de (S)-1'-(6-((2-amino-3-chloropyridin-4-yl)thio)-1,2,4-triazin-3-yl)-1,3-dihydrospiro[indène-2,4'-pipéridin]-1-amine monosuccinate selon l'une quelconque des revendications 1 à 12 pour une utilisation dans le traitement de la croissance cellulaire anormale chez un mammifère, dans laquelle le mammifère est humain.

16. Composé pour une utilisation selon la revendication 15, dans lequel la croissance cellulaire anormale est un cancer.

17. Composé pour une utilisation selon la revendication 16, dans lequel le cancer est sélectionné dans le groupe constitué par : mélanome, leucémies myélomonocytaires juvéniles, neuroblastome, leucémie myéloïde chronique positive au chromosome de Philadelphie, leucémies lymphoblastiques aiguës positives au chromosome de Philadelphie, leucémies myéloïdes aiguës, néoplasmes myéloprolifératifs, cancer du sein, cancer du poumon, cancer du foie, cancer colorectal, cancer de l'œsophage, cancer gastrique, carcinome épidermoïde de la tête et du cou, glioblastome, lymphome anaplasique à grandes cellules, carcinome de la thyroïde, néoplasmes spitoïdes, cancer du poumon non à petites cellules, cancer du côlon, cancer de l'œsophage, cancer du rectum, leucémie myélomonocytaire juvénile, cancer du sein, mélanome et cancer du pancréas, CPNPC ALK-positif, CPNPC ROS1-positif, cancer colorectal à mutation BRAF V600E, tumeurs solides à mutation RAS, tumeurs solides à mutation NF1 et tumeurs solides à mutation BRAF de classe 3.

18. Composé pour une utilisation selon la revendication 17, dans lequel le cancer est un CPNPC ALK-positif.

19. Composé pour une utilisation selon la revendication 17, dans lequel le cancer est un cancer colorectal à mutation BRAF V600E.

20. Composé pour une utilisation selon la revendication 17, dans lequel le cancer est des tumeurs solides à mutation RAS.

21. Composé pour une utilisation selon la revendication 17, dans lequel le cancer est des tumeurs solides à mutation NF1.

22. Composé pour une utilisation selon la revendication 17, dans lequel le cancer est des tumeurs solides à mutation BRAF de classe 3.

23. Composé pour une utilisation selon la revendication 16, dans lequel le cancer est un cancer à mutation KRAS.

24. Composé pour une utilisation selon la revendication 23, dans lequel la mutation KRAS est sélectionnée parmi une mutation KRASG12A, une mutation KRASG12C, une mutation KRASG12D, une mutation KRASG12F, une mutation KRASG12I, une mutation KRASG12L, une mutation KRASG12R, une mutation KRASG12S, une mutation KRASG12V et une mutation KRASG12Y.

25. Composé selon l'une quelconque des revendications 1 à 12 pour une utilisation en combinaison avec un composé thérapeutique supplémentaire.

26. Composé pour une utilisation selon la revendication 25, dans lequel le thérapeutique supplémentaire est sélectionné dans le groupe constitué par lorlatinib, binimiétinib, cétuximab et encorafénib.
